# EUROPÄISCHE PATENTSCHRIFT

(11) **EP 4 119 148 B1**
(45) Veröffentlichungstag und Bekanntmachung des Hinweises auf die Patenterteilung: **02.09.2026**
(21) Anmeldenummer: 22184731.2
(22) Anmeldetag: 13.07.2022
(51) Int. Cl.: A61K 33/38, A61K 47/42, A61K 9/51, A61K 9/10, A61K 9/00, A61P 31/12, A61P 31/04, A61K 47/26, A61K 47/02, A61K 45/06

(54) **SILBERNANOPARTIKEL ZUR VERWENDUNG BEI DER BEHANDLUNG VON INFEKTIONEN DER ATEMWEGE**
SILVER NANOPARTICLES FOR USE IN THE TREATMENT OF RESPIRATORY TRACT INFEKTIONS
NANOPARTICULES D'ARGENT DESTINÉES À ÊTRE UTILISÉES DANS LE TRAITEMENT D'INFEKTIONS DES VOIES RESPIRATOIRES

(30) Priorität: 16.07.2021 EP 21186143
(43) Veröffentlichungstag der Anmeldung: 18.01.2023
(73) Patentinhaber: B+H Solutions GmbH, 73630 Remshalden (DE)
(72) Erfinder: Heinisch, Martin, 73630 Remshalden (DE); Heinisch, Laura, 73630 Remshalden (DE); Sarkar, Subhasish, 73630 Remshalden (DE)
(74) Vertreter: Glück Kritzenberger Patentanwälte PartGmbB

(56) Entgegenhaltungen:
- WO-A1-2012/084072
- LV XIAONAN ET AL: "Inhibitory effect of silver nanomaterials on transmissible virus-induced host cell infections", BIOMATERIALS, ELSEVIER, AMSTERDAM, NL, vol. 35, no. 13, 10 February 2014 (2014-02-10), pages 4195 - 4203, XP028623064, ISSN: 0142-9612, DOI: 10.1016/J.BIOMATERIALS.2014.01.054
- L. KVITEK ET AL: "Effect of Surfactants and Polymers on Stability and Antibacterial Activity of Silver Nanoparticles (NPs)", JOURNAL OF PHYSICAL CHEMISTRY C, vol. 112, no. 15, 17 April 2008 (2008-04-17), pages 5825 - 5834, XP055030297, ISSN: 1932-7447, DOI: 10.1021/jp711616v
- ZACHAR ORON: "Formulations for COVID-19 Early Stage Treatment via Silver Nanoparticles Inhalation Delivery at Home and Hospital", SCIENCEOPEN, 28 March 2020 (2020-03-28), pages 1 - 15, XP055880153, Retrieved from the Internet <URL:https://pdfs.semanticscholar.org/a7ec/11bb12971727cec3bf60d13f48f227ec7f47.pdf?_ga=2.117256581.221819182.1643014224-451394286.1633593488> [retrieved on 20220117], DOI: 10.14293/S2199-1006.1.SOR-.PPHBJEO.v1
- MADURAY KAMINEE ET AL: "Metal Nanoparticles: a Promising Treatment for Viral and Arboviral Infections", BIOLOGICAL TRACE ELEMENT RESEARCH, HUMANA PRESS, CLIFTON, NJ, US, vol. 199, no. 8, 7 October 2020 (2020-10-07), pages 3159 - 3176, XP037489086, ISSN: 0163-4984, [retrieved on 20201007], DOI: 10.1007/S12011-020-02414-2
- B+H SOLUTIONS GMBH ET AL: "Lösungen für Pflanzen und Oberflächen", 23 March 2020 (2020-03-23), pages 1 - 2, XP055880479, Retrieved from the Internet <URL:https://www.protexme.de/media/47/a8/19/1619606630/Technisches%20Datenblatt_AgSept.pdf> [retrieved on 20220118]
- YANG XIAO XI ET AL: "Curcumin modified silver nanoparticles for highly efficient inhibition of respiratory syncytial virus infection", vol. 8, no. 5, 1 January 2016 (2016-01-01), United Kingdom, pages 3040 - 3048, XP055880627, ISSN: 2040-3364, Retrieved from the Internet <URL:https://pubs.rsc.org/en/content/articlepdf/2016/nr/c5nr07918g> DOI: 10.1039/C5NR07918G
- LI YINGHUA ET AL: "Silver Nanoparticle Based Codelivery of Oseltamivir to Inhibit the Activity of the H1N1 Influenza Virus through ROS-Mediated Signaling Pathways", APPLIED MATERIALS & INTERFACES, vol. 8, no. 37, 21 September 2016 (2016-09-21), US, pages 24385 - 24393, XP055880620, ISSN: 1944-8244, Retrieved from the Internet <URL:https://pubs.acs.org/doi/pdf/10.1021/acsami.6b06613> DOI: 10.1021/acsami.6b06613

## Beschreibung

### Technisches Gebiet

Die Erfindung betrifft eine silbernanopartikelhaltige, wässrige Formulierung zur Verwendung als Medikament.

### Stand der Technik

Einer allgemeinen Definition folgend ist "Nanopartikel" eine Bezeichnung für Partikel, die eine Größe im Bereich kleiner als oder gleich 100 nm aufweisen. Die Verwendung der Vorsilbe "Nano" stellt somit, entsprechend der offiziellen Definition nach ISO TC 229, eine Abgrenzung zu Partikel im Sub-Mikrometer Bereich (> 100 nm) dar. Generell ist davon auszugehen, dass Stoffe, die als Nanomaterial bezeichnet werden, veränderte chemische und physikalische Eigenschaften besitzen. Nanometalle zeigen andere Farben als die entsprechenden Metalle, im Falle von Gold beispielsweise die Farbe Rot.

Darüber hinaus ist es wissenschaftlich belegt, dass Nanopartikel eines Stoffes eine erhöhte Oberflächenenergie besitzen. Je kleiner die Partikel sind, desto höher ist deren Oberflächenenergie. In der Konsequenz sind Nanopartikel generell als instabil zu betrachten, da sie aufgrund ihrer hohen Oberflächenenergie leicht zu neuen Verbindungen reagieren bzw. größere, stabilere Aggregate bilden. Für das Beispiel der Nanometalle bedeutet dies, dass selbst die Partikel edler Metalle schnell mit Luftsauerstoff oxidieren sobald die Größe der Partikel im Bereich von Nanometern liegt.

Technologisch nutzbare Nanopartikel erhält man, wenn deren Oberflächen chemisch oder physikalisch geschützt und damit stabilisiert sind. Möglichkeiten der Stabilisierung von Nanopartikeln in Dispersionen sind aus dem Stand der Technik bekannt (vgl. z.B. WO 2008/017176 A1, WO 2009/072911 A1). Es existieren verschiedene Verfahrenswege zur Herstellung von stabilisierten metallischen Nanopartikeln. Die Nanopartikel können z.B. auf Feststoffen geträgert werden. Daneben können Metallnanopartikel mittels Polymeren wie Polyvinylpyrrolidon bei der Herstellung im Polyolprozess stabilisiert werden. Schließlich können Metallnanopartikel durch ein PVD Verfahren (Physical Vapor Deposition) erzeugt werden, bei dem das zugrunde liegende Metall verdampft wird. Zur Stabilisierung der so erzeugten Nanopartikel werden Polymere bzw. Silikone verwendet.

Die antivirale Aktivität von Metallnanopartikeln ist seit längerer Zeit Gegenstand von Untersuchungen. Ein Überblick über dieses Forschungsgebiet findet sich in Maduray et al., ("Metal Nanoparticles: a Promising Treatment for Viral and Arboviral Infections" Biological Trace Element Research 199, 3159-3176 (2021), https://doi.org/10.1007/s12011-020-02414-2). Weiter wurde der Einsatz von Nanosilberpartikeln als antiviral wirkende Substanz gegen SARS-CoV-2 untersucht, nicht aber als Medikament, sondern zur Desinfektion (Jeremiah et al. "Potent antiviral effect of silver nanoparticles on SARS-CoV-2" Biochemical and Biophysical Research Communications, Volume 533, Issue 1, 26 November 2020, S. 195-200, doi: 10.1016/j.bbrc.2020.09.018). In der Studie konnte die antivirale Wirksamkeit von Silbernanopartikeln (AgNPs) belegt werden, welche aber durch die mit den Nanosilberpartikeln verbundenen zytotoxischen Effekte limitiert ist.

LV XIAONAN ET AL: "Inhibitory effect of silver nanomaterials on transmissible virus-induced host cell infections", BIOMATERIALS, ELSEVIER, AMSTERDAM, NL, Bd. 35, Nr. 13, 10. Februar 2014 (2014-02-10), Seiten 4195-4203 ,ISSN: 0142-9612, DOI: 10.1016/J.BIOMATERIALS.2014.01.054 zeigt in vitro antivirale Wirksamkeit von Silbernanopartikeln gegen Coronaviren.

Zachar ("Formulations for COVID-19 Early Stage Treatment via Silver Nanoparticles Inhalation Delivery at Home and Hospital", ScienceOpen Preprints, DOI: 10.14293/S2199-1006.1.SOR-.PPHBJEO.v1) untersuchte die Möglichkeit, eine effektive minimale Hemmkonzentration (MHK) von Silber-Nanopartikeln an verschiedenen Zielorten des Atmungssystems zur Unterdrückung sowohl viraler als auch bakterieller Atemwegsinfektionen zu erhalten. Zu den Anwendungen gehören (i) die Kontrolle lokaler Ausbrüche von COVID-19 durch frühzeitige Behandlung zu Hause und (ii) die Senkung des Risikos einer beatmungsassoziierten Pneumonie (VAP) auf der Intensivstation eines Krankenhauses. Ziel war es, eine First-Line-Interventionsmaßnahme vorzuschlagen, die das Potenzial hat, die Ausbreitung der viralen Infektion im gesamten Atmungssystem zu unterdrücken, wodurch mehr Zeit für eine angemessene Reaktion des Immunsystems zur Verfügung stehen soll und das Risiko einer Verschlimmerung und Ausbreitung der Infektion gesenkt werden soll. Des Weiteren werden die verfügbaren glaubwürdigen Beweise für die Sicherheit beim Menschen durch Inhalation diskutiert, um klinische Studien zu ermöglichen. Basierend auf zuvor veröffentlichten experimentellen Daten zur antiviralen Wirksamkeit von kolloidalem Silber schlägt er eine Modellmethode und -berechnung zur Erreichung der antiviralen MHK von Silberpartikeln an verschiedenen Stellen des Atmungssystems vor, durch: (a) Analyse der von der Nanopartikelgröße abhängigen erforderlichen Konzentration. (b) die Berechnung der erforderlichen Aerosolabgabeeigenschaften. Um die benötigte Dosis zu berechnen, berücksichtigt er Depositionsanteilsverluste und auch den Inhalationszeitanteil des normalen Atemzyklus. Er wertet das unabhängige Targeting von: (i) dem Trachea-Bronchial-Baum (Schleimvolumen von etwa 1 cc) und (ii) den Alveolen (Gesamtschleimvolumen von etwa 10 cc). Es wird kein experimenteller Nachweis erbracht. Es wird gezeigt, dass die Dosierung empfindlich auf die Größe der Silber-Nanopartikel reagiert, wobei 3 nm - 7 nm aufgrund der Berechnungen die optimale Größe ist. Die effektive antibakterielle MHK wird aufgrund der Berechnungen auf 10 µg/ml geschätzt, aber für mehr Sicherheit werden 25 µg/ml als vernünftige Zielkonzentration angegeben, die in der Schleimflüssigkeit der Atemwege erreicht werden sollte. Insbesondere behauptet er, dass unter Verwendung von kolloidalem Silber mit 5 nm großen Partikeln, die Inhalation von Standard-Aerosol-Tropfen mit 5 µm Durchmesser (z. B. unter Verwendung von handelsüblichen Ultraschallverneblern), eine ausreichende MHK erreicht werden kann mit: (i) Ablagerung von insgesamt nur 0,25 cm³ einer Quellkonzentration von 100 ppm (µg/ml) im Bronchialbaum und (ii) Ablagerung von insgesamt 1 cm³ einer Quellkonzentration von 250 ppm (µg/ml) in den Lungenalveolen. Nach Berücksichtigung der Depositionsverluste und aufgrund der Tatsache, dass die aktive Inhalationszeit nur etwa 1/3 des Atemzyklus beträgt, stellt er aufgrund der Berechnungen fest, dass mit diesen Aerosolierungsdosen eine praktisch wirksame MHK erreicht werden kann: (a) für die oberen Atemwege und den Bronchialbaum mit 2 cc einer 100 µg/ml kolloidalen Silberquelle, während (b) für die Lungenalveolen 6 cc einer 200 µg/ml kolloidalen Silberquelle verwendet werden. Dies würde sich um den Faktor 3 verringern, wenn ein atembetätigter Ultraschallvernebler verwendet wird. Er schlussfolgert aus den Berechnungen, dass eine wirksame MHK sowohl im Bronchialbaum als auch in den Alveolen erreicht werden kann (obwohl die spezifische Aerosolverabreichung unterschiedlich sein kann). Da Atemwegsinfektionen am häufigsten in den oberen Atemwegen beginnen, schlägt er vor, die vorgestellte Methode am besten frühzeitig als First-Line-Behandlung einzusetzen, um das Fortschreiten der Infektion zu unterdrücken.

Coronaviren sind unter Säugetieren und Vögeln weit verbreitet. Sie verursachen beim Menschen vorwiegend milde Erkältungskrankheiten, können aber mitunter auch schwere Lungenentzündungen hervorrufen. Anfang 2020 wurde das neue Beta-Coronavirus SARS-CoV-2 (severe acute respiratory syndrome coronavirus type 2) als Auslöser der Krankheit COVID-19 identifiziert.

SARS-CoV-2 verwendet das Enzym ACE-2 als Rezeptor, um in die Wirtszellen zu gelangen. Eine hohe ACE-2-Dichte besteht insbesondere im Atemwegstrakt, weshalb SARS-CoV-2 sehr häufig Atemwegsinfektionen verursacht. Meist in der zweiten Krankheitswoche kann sich eine Pneumonie entwickeln, die in ein beatmungspflichtiges ARDS (Acute Respiratory Distress Syndrome) fortschreiten kann, das unter Umständen eine Sauerstoffaufsättigung des Blutes außerhalb des Körpers (ECMO) erforderlich macht.

Nur ein Teil der COVID-19-Erkrankungen verläuft schwer. Im Zentrum der Behandlung der Infektion stehen die optimalen unterstützenden Maßnahmen entsprechend der Schwere des Krankheitsbildes (z. B. Sauerstoffgabe, Ausgleich des Flüssigkeitshaushaltes, ggf. Antibiotikagabe zur Behandlung von bakteriellen Ko-Infektionen) sowie die Behandlung von relevanten Grunderkrankungen.

Viele verschiedene spezifische Therapieansätze (direkt antiviral wirksam, immunmodulatorisch wirksam) wurden und werden im Verlauf der Pandemie durch SARS-CoV-2 in Studien untersucht. Als immunmodulatorisch wirksames Arzneimittel wird Dexamethason bei Patienten mit einer Infektion durch SARS-CoV-2 angewendet, die eine zusätzliche Sauerstoffzufuhr oder künstliche Beatmung erfordert.

Als direkt antiviral wirksames Arzneimittel erhielt Remdesivir (Veklury^{®}) am 3. Juli 2020 eine bedingte Zulassung durch die EMA zur Anwendung bei Patienten mit einer Pneumonie, die eine zusätzliche Sauerstoffzufuhr erfordert (Low- oder High-Flow-Sauerstofftherapie oder nicht-invasive Beatmung) in früher Phase der Erkrankung. Ebenfalls direkt antiviral wirksam sind monoklonale Antikörper. Zurzeit befinden sich verschiedene monoklonale Antikörper in Untersuchung durch klinische Studien.

Da die SARS-CoV-2 Pandemie eine weiter andauernde gesundheitliche Bedrohung für die gesamte Menschheit darstellt, besteht weiterhin ein Bedarf an Medikamenten zur Behandlung von Virus-basierten Erkrankungen des Respirationstraktes und insbesondere zur Behandlung von COVID-19-Erkrankungen.

### Darstellung der Erfindung

Der Erfindung, wie sie in den Ansprüchen gekennzeichnet ist, liegt die Aufgabe zugrunde, ein Medikament zur Behandlung von Virus-basierten Erkrankungen des Respirationstraktes zur Verfügung zu stellen, insbesondere zur Behandlung von COVID-19.

Diese Aufgabe wird erfindungsgemäß durch die silbernanopartikelhaltige Formulierung zur Verwendung als Medikament bei der Prävention und/oder der Behandlung von mikrobiellen Erkrankungen und/oder Virus-basierten Erkrankungen des Respirationstraktes gemäß Anspruch 1 gelöst. Weitere vorteilhafte Details, Aspekte und Ausgestaltungen der vorliegenden Erfindung ergeben sich aus den abhängigen Ansprüchen, der Beschreibung, den Beispielen und den Figuren.

Die vorliegende Erfindung stellt eine Formulierung zur Verwendung als Medikament bei der Prävention und/oder der Behandlung von mikrobiellen Erkrankungen und/oder Virus-basierten Erkrankungen des Respirationstraktes zur Verfügung. Die Formulierung umfasst in Wasser 0,02 Gew.-% bis 0,5 Gew.-% Silbernanopartikel mit einer mittleren Partikelgröße von 10 nm bis 20 nm in Wasser und wenigstens zwei verschiedene Stabilisatoren aus der Gruppe der nichtionischen Tenside. Das Gewichtsverhältnis Silbernanopartikel zur Summe der Stabilisatoren liegt im Bereich von 10:2 bis zu 10:50. Die wenigstens zwei verschiedenen Stabilisatoren sind ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester, Polyoxypropylen-tri-alkylsäureester und deren Gemische.

Im vorliegenden Text beziehen sich sämtliche Angaben von Anteilen in Gew.-% auf das Gewicht der gesamten Formulierung als 100%-Basis.

Als Wasser-Basis wird für die Formulierung typischerweise destilliertes Wasser oder eine physiologische Lösung, d. h. eine 0,9%ige Natriumchlorid-Lösung verwendet.

Die Formulierung enthält zumindest zwei Stabilisatoren ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester und Polyoxypropylen-tri-alkylsäureester. Bei diesen Stabilisatoren handelt es sich um Verbindungen mit grenzflächenaktiven Eigenschaften aus der Gruppe der nichtionischen Tenside, die bei Raumtemperatur in flüssiger Form vorliegen. Nichtionische Tenside sind grenzflächenaktive chemische Komponenten, die ungeladene polare und unpolare Bereiche in einem Molekül aufweisen. Darüber hinaus weisen nichtionische Tenside keine dissoziierten funktionellen Gruppen auf.

Diese Stabilisatoren umhüllen die Silbernanopartikel in der wässrigen Lösung bei den unterschiedlichen Konzentrationen und stabilisieren die Nanopartikel in der Formulierung vor der Verabreichung, sie führen aber auch dazu, dass die Silbernanopartikel im Körper an den Zielort transportiert werden können und dort optimale Wirkung entfalten können. Sie führen, ohne an eine wissenschaftliche Erklärung gebunden zu sein, auch dazu, dass der Hauptwirkstoff, der Silbernanopartikel, über längere Zeit wirksam ist, und gewissermaßen eine verzögerte Freisetzung des Wirkstoffs gewährleistet wird.

Die erfindungsgemäß zur Verwendung als Medikament bei der Prävention und/oder der Behandlung von mikrobiellen Erkrankungen und/oder Virus-basierten Erkrankungen vorgesehene silbernanopartikelhaltige Formulierung enthält mit anderen Worten dispersionsstabilisierte Silbernanopartikel, die nicht zu größeren Agglomeraten aggregieren können, da die verwendeten Stabilisatoren im Temperaturbereich von 0°C bis 240°C flüssig sind. Aus dem Stand der Technik sind auch viele Silbernanopartikelprodukte bekannt, die als trockene Pulver angeboten werden, die aber aufgrund ihrer Agglomerationsneigung während Transport und Lagerung zur Dispergierung in organischen Lösungsmitteln, wie Acryl- und Epoxidharzen, nur unter hohem mechanischen Energieeintrag und dann auch nur unvollständig dispergiert werden können. Diese Silbernanopartikelprodukte sind für die Verwendung als Medikament allein schon deswegen nicht geeignet.

Bei den zumindest zwei Stabilisatoren in der erfindungsgemäß zur Verwendung als Medikament bei der Prävention und/oder der Behandlung von mikrobiellen Erkrankungen und/oder Virus-basierten Erkrankungen vorgesehenen Formulierung kann es sich um verschiedene Stabilisatoren einer der genannten Klassen chemischer Verbindungen handeln oder um Stabilisatoren verschiedener Verbindungsklassen. Bei einer Kombination von drei verschiedenen Stabilisatoren können also beispielsweise drei verschiedene Polyoxyethylen-mono-alkylsäureester eingesetzt werden, es können aber beispielsweise auch zwei verschiedene Polyoxyethylen-mono-alkylsäureester und ein Polyoxypropylen-mono-alkylsäureester oder beispielsweise ein Polyoxypropylen-di-alkylsäureester, ein Polyoxyethylen-tri-alkylsäureester und ein Polyoxypropylen-tri-alkylsäureester verwendet werden. Es ist jede beliebige Kombination dieser nichtionischen Tenside möglich.

Überraschenderweise hat sich gezeigt, dass die oben angegebene silbernanopartikelhaltige, wässrige Formulierung, die bisher aus dem Stand der Technik lediglich zur Oberflächenbehandlung von Holz, in Imprägnierölen für Luftfilter, als textile Färbelösung, zur Wachstumsförderung von Pflanzen, zur Herstellung antimikrobieller Oberflächen, zur Herstellung von Beschichtungen für Vlies-/Folienlaminate, zur Herstellung von Beschichtungen für textile Dekorstoffe, in Lacken und Klebstoffen, in Silikonen, in thermoplastischen Kunststoffen, in Wood Plastic Composites (WPC), in Polyolefin Formkörpern, in PVC und Plastisolen und zur Herstellung von Fasern bekannt ist, ausgezeichnete Effekte beim Einsatz als Medikament bei der Prävention und/oder der Behandlung von mikrobiellen Erkrankungen und/oder Virus-basierten Erkrankungen des Respirationstraktes zeigt.

Gemäß einer besonders bevorzugten Ausführungsform der vorliegenden Erfindung wird die silbernanopartikelhaltige, wässrige Formulierung bei der Behandlung von Virus-basierten Erkrankungen des Respirationstraktes eingesetzt. Der Einsatz der Formulierung als Medikament bei der Behandlung der genannten Krankheiten zeigt besonders positive Effekte im Hinblick auf den Zeitraum bis zur Genesung und auch im Hinblick auf die Vollständigkeit der Genesung. Es zeigt sich auch, dass, wohl wegen der vorhandenen Stabilisierung, derartige Formulierungen auch über einen größeren Konzentrationsbereich sicher verabreicht werden können, entweder intravenös oder über Inhalation, und zwar ohne toxische Wirkung zu zeigen.

Die vorgeschlagene Formulierung wird intravenös oder per Inhalation beim Menschen angewendet. Sie kann kurativ wie auch präventiv eingesetzt werden. Vorzugsweise wird sie intravenös eingesetzt bei schwer erkrankten Patienten, besonders gute Resultate können nachgewiesen werden bei terminalen oder nahezu terminalen COVID-19-Patienten.

In nachfolgend noch näher erläuterten klinischen Studien hat sich gezeigt, dass die Verabreichung von wasserdispergierten Silber-Nanopartikeln (AgNP) mit einer Größe von insbesondere im Bereich von 10 nm bis 20 nm mit Hilfe von Bronchodilatatoren in die Lunge durch Vernebelung insbesondere bei leichten Fällen von Virus-basierten Erkrankungen des Respirationstraktes zu einer schnelleren Heilung führt.

Bei mittelschweren bis schweren Fällen kann die systemische Verabreichung von Silber-Nanopartikeln die Sterblichkeit bei Patienten mit Virus-basierten Erkrankungen des Respirationstraktes, insbesondere bei COVID-Patienten, verringern.

Aufgrund der starken antiviralen Aktivität von Silber wird vermutet, dass zumindest ein Teil der Viren über das respiratorische Epithel direkt abgetötet wird. Bei systemischer Anwendung ist zu vermuten, dass eine tödliche Virämie im Blut reduziert wird.

Es ist bekannt, dass es bei einer COVID-Pneumonie zu einer Störung des Immunsystems kommt, wodurch eine übermäßige Produktion von entzündlichen Zytokinen verursacht wird. Da Silbernanopartikel in der Lage sind, die Produktion von entzündlichen Zytokinen zu verringern und gleichzeitig die Bildung entzündungshemmender Zytokine zu erhöhen, könnte dieser Mechanismus zu einer geringeren Sterblichkeit führen.

Ebenso kann vermutet werden, dass die aus den AgNPs freigesetzten Ag⁺-Ionen eine Veränderung des pH-Wertes des respiratorischen Epithels verursachen, was zu einer für die Viren lebensfeindlichen Umgebung führt. Experimentelle Ergebnisse deuten darauf hin, dass es eine direkte, vom niedrigen pH-Wert abhängige Fusionsaktivierung von Coronaviren während des Eintritts in die Wirtszellen gibt. Aufgrund der dadurch reduzierten Viruslast im respiratorischen Epithel sowie in anderen Körperflüssigkeiten wird die Wahrscheinlichkeit einer Übertragung von der infizierten Person auf gesunde Personen verringert. Bekanntermaßen stellt Husten oder Niesen mit Ausstoß von virusbeladenen Tröpfchen eine der Hauptansteckungsrouten dar.

Bekanntlich werden Ag⁺-Ionen aus den Silbernanopartikeln herausgelöst, wodurch es zu einer Hemmung der Bindung des Virus an das respiratorische Epithel kommen könnte. Aufgrund der langen Halbwertszeit der Silber-Nanopartikel kann daher als weiterer positiver Effekt eine verringerte Wahrscheinlichkeit von bakteriellen Sekundärinfektionen, wie sie bei schweren Fällen von COVID-19 häufig auftreten, vermutet werden.

Besonders bevorzugt handelt es sich bei der zur Verwendung als Medikament bei der Prävention und/oder der Behandlung von mikrobiellen Erkrankungen und/oder Virus-basierten Erkrankungen vorgesehenen Formulierung um eine silbernanopartikelhaltige, disperse, wässrige Formulierung.

Besonders bevorzugt wird die silbernanopartikelhaltige, wässrige Formulierung bei akuter Rhinits, chronischer Rhinitis, Paryngitis, Herpangina, Angina lateralis, Tonsilitis, Laryngitis, Tracheitis, akuter Bronchiolitis, chronischer Bronchiolitis, akuter Bronchitis, chronischer Bronchitis, Aveolitis, Pneumonie, akuter Sinusitis, chronischef Sinusitis, Gingivastomatitis herpetica, Aphtosis herpetica, Herpes nasalis, Adenovirus-Pharyngokonjunktivitis, infektiöser Mononukleose, akutem Asthma, chronischem Asthma, COPD (chronic obstructive pulmonary disease), Kehlkopfdiphterie, Otitis media, Entzündung der Eustachischen Röhre und/oder bei COVID-19 verwendet.

Besonders positive Effekte im Hinblick auf die Länge des Zeitraums der Genesung wie auch im Hinblick auf die Vollständigkeit der Genesung ergeben sich, wenn es sich um eine Kombination von zwei oder mehreren Virus-basierten Erkrankungen des Respirationstraktes handelt, ggf. kombiniert mit bakteriellen Sekundärinfektionen.

Zunehmend positive Effekte zeigen sich, wenn es sich bei dem Virus um ein Virus der Familie Picornaviridae, Orthomyxoviridae, Paramyxoviriae, Coronaviridae, Adenoviridae und/oder Herpesviridae handelt, um ein Virus der Gattung Rhinovirus, Enterovirus, Influenzavirus, Pneumovirus, Metapneumovirus, Betacoronavirus, Mastadenovirus, Simplexvirus und/oder Varizellovirus, um ein Virus der Art Rhinivirus, Cosackievirus-1, Cosackievirus-2, Echovirus, Poliovirus, Influenzavirus A, Influenzavirus B, Parainfluenzavirus, Respiratorisches Synzitialvirus (RSV), Humanes Metapneumovirus, severe acute respiratory syndrome-related Coronavirus (SARSr-CoV), humanes Adenovirus A, humanes Adenovirus B, humanes Adenovirus C, humanes Adenovirus D, humanes Adenovirus E, humanes Adenovirus F, humanes Herpesvirus 4 (HHV-4), Herpes simplex Virus 1 (HSV-1), Herpes simplex Virus 2 (HSV-2) und/oder Varizella zoster Virus (VZV) und insbesondere um ein Virus der Unterart severe acute respiratory syndrome Coronavirus 2 (SARS-CoV-2) handelt.

Besonders bevorzugt ist die silbernanopartikelhaltige, wässrige Formulierung zur intravenösen Verabreichung oder zur Verabreichung per Inhalation als Aerosol geeignet. In den bisher durchgeführten Studien haben sich diese beiden Darreichungsformen als leicht praktizierbar bei sehr guter Effektivität herausgestellt.

Bevorzugt liegt die silbernanopartikelhaltige, wässrige Formulierung in Kombination mit mindestens einem weiteren pharmazeutischen Wirkstoff und/oder mindestens einem weiteren nicht-pharmazeutischen Wirkstoff vor. Die Einsatzbandbreite der Formulierung wird dadurch wesentlich erhöht.

Besonders bevorzugt ist der mindestens eine weitere pharmazeutische Wirkstoff ausgewählt aus der Gruppe bestehend aus Virostatika, Sympathomimetika, Proteinen, Peptiden, Nukleinsäuren und immunsuppressiven Wirkstoffen. Ebenfalls bevorzugt sind Ausführungsformen, bei denen der mindestens eine weitere nicht-pharmazeutische Wirkstoff ausgewählt ist aus der Gruppe bestehend aus pharmazeutisch verträglichen anorganischen oder organischen Säuren oder Basen, Polymeren, Copolymeren, Block-Copolymeren, Einfachzucker, Mehrfachzucker, ionischen und nicht-ionischen Tensiden oder Lipiden sowie Mischungen hiervon, Albumin, Transferrin und DNA-Reparatur-Proteinen, wie Kinase-Inhibitoren.

Besonders bevorzugt sind in der Formulierung Stabilisatoren in Form einer Kombination nichtionischer Tenside aus zwei unterschiedlichen der Verbindungsklassen Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester und Polyoxypropylen-tri-alkylsäureester enthalten.

Gemäß einer besonders bevorzugten Ausführungsform sind die Stabilisatoren ausgewählt aus der Gruppe bestehend aus Polyoxyethylen-Sorbitan-Monolaurat, Polyoxyethylen-Sorbitan-Monopalmitat, Polyoxyethylen-Sorbitan-Monostearat, Polyoxyethylen-Sorbitan-Monooleat, Polyoxyethylen-Sorbitan-Tristearat, Polyoxyethylen-Glyceryl-Trioleat, Polyoxyethylen-Glyceryl-Monolaurat, Polyoxyethylen-Glyceryl-Monooleat, Polyoxyethylen-Glyceryl-Monostearat, Polyoxyethylen-Glyceryl-Monoricinoleat, Rizinusöl, hydriertes Rizinusöl, Sojabohnenöl und deren Gemische.

Die Stabilisatoren sind vielfach nicht unter ihrem Chemikaliennamen bekannt sondern unter ihrer jeweiligen Handelsbezeichnung. Bevorzugte Stabilisatoren sind Tween20TM, Tween40^{™}, Tween60^{™}, Tween8^{™}, Polysorbat^{™}, Tagat TO^{™}, Tagat TO V^{™}, Tagat L2^{™}, Tagat S2^{™}, Tagat R40^{™}, Triton X 100^{™}, Hydrogenated Castoroil, PEG 20 Glycerylstearat, PEG 20 Glyceryllaurat, PEG 40 Castoroil, PEG 25 Glyceryltrioleat, Newcol^{™}, Montane^{™}, Lonzest^{™}, Liposorb^{™}, Nonion^{™}, Kuplur^{™}, Ionet^{™}, Kemotan^{™}, Grillosan^{™}, Ethylan^{™}, Glycomul^{™}, Emsorb^{™}, Disponil^{™}, Amisol^{™}, Armotan^{™}, Sorbax^{™}, Sorbitan^{™}, Span^{™} und Tego Pearl^{™}.

Ganz besonders bevorzugt werden die Stablisatoren Tween20^{™}, auch bezeichnet als Polysorbat 20 oder Polyoxyethylen(20)-sorbitan-monolaurat, und PEG 25 Glyceryltrioleat, auch bezeichnet als PEG 25 Trioleat oder 1-[2,3-bis(2-oxopropoxy)propoxy]propan-2-on. Insbesondere bevorzugt wird ein Gemisch dieser beiden Stabilisatoren und beste Ergebnisse werden erzielt, wenn die beiden Stabilisatoren im Verhältnis 1:1 enthalten sind.

Obige Liste von Stabilisatoren ist nicht vollständig, da verschiedene Hersteller gleiche oder ähnliche Produkte unter anderen Namen vermarkten bzw. neue nichtionische Tenside der oben genannten Verbindungsklassen in der Zukunft synthetisiert werden und ebenfalls verwendet werden können.

Bevorzugt sind die zumindest zwei Stabilisatoren in der Formulierung in einem Mengenverhältnis im Bereich von 1:1 bis 2:1 anwesend.

Da zumindest zwei nichtionischen Tenside als Stabilisatoren für das Nanosilber verwendet werden, besteht ein Mengenzusammenhang zwischen den Konzentrationen der Stabilisatoren und des Silbers. Die Formulierung weist ein Mengenverhältnis Silbernanopartikel zu Stabilisatoren im Bereich von 10:2 bis 10:50, bevorzugt 10:5 bis zu 10:10, auf. Bevorzugt beträgt das Mengenverhältnis Silber zu Stabilisatoren von 10:5 bis 10:20, besonders bevorzugt von 10:6 bis 10:10. Unter dem "Mengenverhältnis Silber zu Stabilisator" ist das "Mengenverhältnis Silber zur Summe der anwesenden Stabilisatoren" zu verstehen. Bei Verwendung der bevorzugten Mengenverhältnisse erhält man besonders stabile Dispersionen von Silbernanopartikeln.

Die Silbernanopartikel weisen bevorzugt eine Größenverteilung auf, bei der 90 Gew.-% der Teile kleiner sind als 25 nm oder kleiner als 20 nm.

Besonders bevorzugt sind Silbernanopartikel, die eine Verteilung aufweisen, dass 99 Gew.-% kleiner sind als 20nm.

Alternativ oder zusätzlich sind Silbernanopartikel mit einer Verteilung bevorzugt, bei der 66 Gew.-% der Teile kleiner sind als 25 nm, vorzugsweise kleiner sind als 20 nm, insbesondere vorzugsweise kleiner sind als 18 nm, und/oder bei der 33 Gew.-% der Teile kleiner sind als 20 nm, vorzugsweise kleiner sind als 15 nm, insbesondere vorzugsweise kleiner sind als 10 nm.

Dabei wird die Partikelgrössen-Verteilung zusammen mit dem Mittelwert der Grösse (Durchmesser) vorzugsweise unter Einsatz einer elektronenmikroskopischen Analyse bestimmt:
TEM-Gitter (400 mesh, beschichtet mit 8 nm Kohlenstoff) werden für 20 s auf einem 10 ml Tropfen einer Silbernanopartikelsuspension, die 1:90 mit nanoreinem Wasser verdünnt wurde, inkubiert. Anschließend wird die überschüssige Suspensionsflüssigkeit mit einem Filterpapier abgesaugt und die Gitter werden im Gerät abgebildet. Bilder mit einer Größe von 1024 x 1024 Pixel und einer Pixelgröße von 0,376 nm werden mit der Bildverarbeitungssoftware IPS Version 1.123 (Visometrics, Konstanz, Deutschland) ausgewertet, um um die Partikelgröße der Silber-Nanopartikel in der Suspension zu bestimmen. Insgesamt werden wenigstens 500 Partikel, vorzugsweise 500 bis 1000 Partikel analysiert. Diese Messergebnisse werden als Histogramm und Dichteplot unter Verwendung der Software R (http://www.r-project.org) dargestellt und gleichzeitig werden der Mittelwert (mittlere Partikelgösse), die Standardabweichung und ihre robusten Gegenstücke (Median und MAD) der Größenmessung ermittelt.

Erfindungsgemäß weisen die Silbernanopartikel eine mittlere Partikelgröße von 10 nm bis 20 nm, besonders bevorzugt von 15 bis 20 nm, auf.

Die Formulierung ist flüssig und enthält bevorzugt ansonsten keinerlei feste Nebenbestandteile, die die Möglichkeiten der weiteren Verwendung einschränken würden. Dies gilt für das als Ausgangsformulierung verwendete Konzentrat wie auch für die Formulierung nach Verdünnung des Konzentrats mit Wasser im Verhältnis 1:100.

Es können auch mehr als zwei Stabilisatoren in der Formulierung anwesend sein. In diesem Fall liegt der Gehalt eines ersten Stabilisators im Bereich von 30 Gew.-% bis 90 Gew.-%, bevorzugt zwischen 40 Gew.-% bis 60 Gew.-%, besonders bevorzugt zwischen 45 Gew.-% bis 55 Gew.-%. Der verbleibende Gewichtsanteil bis 100 Prozent teilt sich unter die weiteren in Kombination verwendeten Stabilisatoren auf, die sich ihrerseits in Mengenanteilen von 0 bis 100 Gew.-% aufteilen. Die hier genannten Gew.-%-Angaben beziehen sich somit in Abweichung zu sonstigen Angaben auf das Gesamtgewicht an Stabilisatoren als 100%-Basis. Besonders bevorzugt sind in der Formulierung ein oder mehrere Stabilisatoren ausgewählt aus der Gruppe bestehend aus Tagat TO V^{™}, Tween20^{™}, Tween80^{™}, PEG 25 Glyceryltrioleat und Tagat L2^{™} anwesend. Unter Verwendung dieser Stabilisatoren werden besonders stabile und universell einsetzbare Dispersionen wirksam als Medikamente erhalten.

Gemäß einer ganz besonders bevorzugten Ausführungsform der vorliegenden Erfindung ist in der Formulierung als Stabilisator ein Gemisch von PEG 25 Glyceryltrioleat und Tween20^{™} anwesend. Insbesondere bevorzugt sind Formulierungen, in denen das Mengenverhältnis PEG 25 Glyceryltrioleat zu Tween20^{™}im Bereich von 1:2 bis zu 2:1 liegt und ganz besonders bevorzugt sind Formulierungen, in denen das Mengenverhältnis PEG 25 Glyceryltrioleat zu Tween20^{™}ungefähr 1:1 beträgt.

Bekanntermaßen hängt die Wirksamkeit von Medikamenten ganz allgemein extrem stark von der Dosierung und damit von der Konzentration des Wirkstoffes in dem verabreichten Medikament ab. Eine zu geringe Konzentration an Wirkstoff geht mit einem mangelhaften Effekt bei der Behandlung von Patienten mit dem Medikament einher. Eine zu hohe Konzentration an Wirkstoff führt in den meisten Fällen zu einer Vergiftung des Patienten. In den bisher durchgeführten Studien wurde ermittelt, dass der erfindungsgemäße Anteil von 0,02 Gew.-% bis 0,5 Gew.-% Silbernanopartikel in der wässrigen Formulierung diese Bedingungen hinreichend erfüllt.

Erfindungsgemäß sind die Silbernanopartikel in einem Anteil von 0,02 Gew.-% bis 0,5 Gew.-%, bevorzugt in einem Anteil von 0,03 Gew.-% bis 0,4 Gew.-%, insbesondere bevorzugt in einem Anteil im Bereich von 0,04 Gew.-% bis 0,3 Gew.-%, ganz besonders bevorzugt in einem Anteil im Bereich von 0,05 Gew.-% bis 0,2 Gew.-% in der Formulierung enthalten. Mit enger werdenden Bereichen der Konzentration an Silbernanopartikel nehmen die positiven Eigenschaften der als Medikament verwendeten Formulierung immer stärker zu. Als ideal hat sich der in den Studien eingesetzte Anteil von 0,1 Gew.-% Silbernanopartikel in der Formulierung gezeigt.

Gemäß einer weiteren bevorzugten Ausführungsform enthält die silbernanopartikelhaltige, disperse, wässrige Formulierung pro 0,1 Gew.-% Silbernanopartikel Ammoniumnitrat in einem Anteil von 0,025 Gew.-% bis 2,0 Gew.-%, bevorzugt 0,05 Gew.-% bis 1,0 Gew.-%, besonders bevorzugt rund 0,075 Gew.-%.

Bezüglich der Partikelgröße sei nochmals auf die eingangs formulierte Definition verwiesen, wonach die Silbernanopartikel eine Partikelgröße von weniger als 100 nm aufweisen. In der Formulierung liegen die Silbernanopartikel in einer Partikelgröße von 10 nm bis 20 nm, besonders bevorzugt 15 nm bis 20 nm, vor. Die Morphologie der Silbernanoteilchen kann dabei Formen von Dreiecken, Kuben, Sphären, Stäben oder Plättchen aufweisen.

Gemäß einer weiteren, besonders bevorzugten Ausführungsform weisen die Silbernanopartikel eine Größenverteilung auf, bei der 99 Gew.-% der Silbernanopartikel kleiner sind als 30 nm, vorzugsweise kleiner sind als 20 nm. Bevorzugt sind Ausführungsformen, bei denen 90 Gew.-% der Silbernanopartikel kleiner sind als 20 nm. Außerdem sind Ausführungsformen bevorzugt, bei denen 66 Gew.-% der Silbernanopartikel kleiner sind als 18 nm. Schließlich sind auch Ausführungsformen bevorzugt, bei denen 33 Gew.-% der Silbernanopartikel kleiner sind als 15 nm, vorzugsweise kleiner sind als 10 nm.

Die zur Verwendung als Medikament bei der Prävention und/oder der Behandlung von mikrobiellen Erkrankungen und/oder Virus-basierten Erkrankungen, vorgesehene Formulierung kann wie folgt hergestellt werden:
a) Bereitstellen eines silbernanopartikelhaltigen, wässrigen Konzentrats, wobei das Konzentrat 1 Gew.-% bis 60 Gew.-% Silbernanopartikel mit einer mittleren Partikelgröße von 10 nm bis 20 nm und zumindest zwei Stabilisatoren aus der Gruppe der nichtionischen Tenside umfasst, wobei das Mengenverhältnis Silbernanopartikel zu Stabilisator im Bereich von 10:2 bis zu 10:50 liegt, wobei die zumindest zwei Stabilisatoren ausgewählt sind aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester, Polyoxypropylen-tri-alkylsäureester und deren Gemische
b) Verdünnen des in Schritt a) bereitgestellten Konzentrats mit Wasser in einem Verhältnis von 1:100 Vol%.

Verfahren zur Herstellung einer silbernanopartikelhaltigen, dispersen, wässrigen Konzentrats, wobei das Konzentrat 1 Gew.-% bis 60 Gew.-% Silbernanopartikel und zumindest zwei Stabilisatoren aus der Gruppe der nichtionischen Tenside umfasst, wobei das Mengenverhältnis Silbernanopartikel zu Stabilisator im Bereich von 10:2 bis zu 10:50 liegt, wobei die zumindest zwei Stabilisatoren ausgewählt sind aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester, Polyoxypropylen-tri-alkylsäureester und deren Gemische, sind in der EP 2 515 660 B1 ausführlich beschrieben.

Auf die EP 2 515 660 B1 wird hiermit explizit Bezug genommen und auf den Inhalt des Dokumentes im Hinblick auf die Herstellung einer silbernanopartikelhaltigen, dispersen, wässrigen Ausgangsformulierung vor der Verdünnung mit Wasser im Verhältnis 1: 100, also im Hinblick auf die Herstellung eines Konzentrats mit 1 Gew.-% bis 60 Gew.-% Silbernanopartikel mit einer mittleren Partikelgröße von 10 nm bis 20 nm und zumindest zwei Stabilisatoren aus der Gruppe der nichtionischen Tenside, wobei das Mengenverhältnis Silbernanopartikel zu Stabilisator im Bereich von 10:2 bis zu 10:50 liegt, wobei die zumindest zwei Stabilisatoren ausgewählt sind aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester, Polyoxypropylen-tri-alkylsäureester und deren Gemische.

Ein Verfahren zur Herstellung der oben beschriebenen silbernanopartikelhaltigen, dispersen, wässrigen Ausgangsformulierung vor deren Verdünnung mit Wasser im Verhältnis 1:100 umfasst die Schritte:
- Bereitstellen eines Silbersalzes,
- Bereitstellen von zumindest zwei Stabilisatoren ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester, Polyoxypropylen-tri-alkylsäureester und der Gemischen,
- Bereitstellen eines Reduktionsmittels,
- Bereitstellen des Lösungsmittels Wasser,
- Herstellen einer Lösung von Silbersalz, Stabilisator und Reduktionsmittel,
- Zugabe einer Base zu der Lösung, wobei die Zugabe der Base kontinuierlich über einen Zeitraum von 5 h bis 48 h derart erfolgt, dass der pH-Wert der Formulierung zwischen 0 und 6 liegt.

Durch dieses Herstellungsverfahren wird eine Ausgangsformulierung mit einer sehr engen Verteilung der Partikelgrößen der Nanopartikel erhalten.

Bevorzugt erfolgt die Zugabe der Base kontinuierlich über einen Zeitraum von 9 h bis 30 h. Auf diese Weise wird eine Ausgangsformulierung mit einer besonders engen Verteilung der Partikelgrößen der Nanopartikel erhalten.

Das beschriebene Herstellungsverfahren ist ein reduktiver chemischer Prozess. Demnach werden die Silberpartikel durch chemische Reduktion aus ihren Salzen hergestellt. Generell kann zur Herstellung der Silbernanopartikel jedes beliebige chemische oder physikalische Reduktionsmittel verwendet werden. Unter physikalischen Reduktionsmitteln ist dabei eine Temperaturerhöhung oder Bestrahlung mit Licht zu verstehen. Vorteilhaft ist die Verwendung eines chemischen Reduktionsmittels, da hier Stoffumsätze von 100 Prozent und sehr hohe Reaktionsgeschwindigkeiten erzielt werden können.

Es hat sich gezeigt, dass bei Anwesenheit von zumindest zwei Stabilisatoren aus der Gruppe Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester und Polyoxypropylen-tri-alkylsäureester sehr starke Reduktionsmittel verwendet werden können, was zu einer erhöhten Reaktionsgeschwindigkeit führt, ohne gleichzeitige Gefahr der Bildung eines größeren Anteils großer, unerwünschter Silberpartikel. Unter den chemischen Reduktionsmitteln sind solche bevorzugt, die keine in der Reaktionsmischung verbleibenden Reaktionsnebenprodukte, wie etwa die entsprechende oxidierte Form des jeweiligen Reduktionsmittel, erzeugen, welche die Qualität der silbernanopartikelhaltigen, dispersen, wässrigen Ausgangsformulierung mindern würden. Bevorzugt wird daher ein Reduktionsmittel eingesetzt, das mit den Silberionen des Silbersalzes unter Bildung von elementarem Silber und ansonsten überwiegend gasförmigen Reaktionsprodukten reagiert.

Besonders bevorzugt werden Reduktionsmittel, die in ihrer oxidierten Form als gasförmiger Stoff die Reaktionslösung verlassen können, wie etwa Hydrazinhydrat. Selbstverständlich können die Silbernanopartikel ebenso mit jedem beliebigen anderen Reduktionsmittel erhalten werden.

Zur Herstellung der Ausgangsformulierung können beliebige Silbersalze verwendet werden. Aus Gründen der Praktikabilität hängt die Wahl des Silbersalzes von dem eingesetzten Lösungsmittel ab, da unterschiedliche Salze unterschiedliche Lösungseigenschaften besitzen. Bevorzugt wird immer dasjenige Silbersalz, das im jeweiligen Lösungsmittel die beste Löslichkeit besitzt. **In** den nachfolgenden Beispielen sind einige Lösungsmittel und das jeweilig geeignete Silbersalz beschrieben. Das oben beschriebene Herstellungsverfahren ist aber nicht auf die genannten Beispiele von Silbersalz/Lösungsmittel-Paarungen beschränkt.

Die reduktive Herstellung von Silber kann als ein Paar von Redox-Gleichungen formuliert werden. Die erste Teilgleichung ist die Reduktionsgleichung, nach der das Silberkation aus dem Silbersalz zum Elementsilber reduziert wird. Die zweite Teilgleichung beschreibt den entsprechenden oxidativen Vorgang für die Oxidation des Reduktionsmittel zum korrespondierenden Oxidationsprodukt, das idealerweise in gasförmigem Zustand die Reaktionslösung verlässt. Allen Reduktionsmitteln ist gemeinsam, dass je übertragenem Elektron ein Proton entsteht. Dieses Proton trägt dazu bei, dass der pH-Wert der gesamten Reaktionslösung sehr stark fällt. Die Abnahme des pH-Wertes ist dafür verantwortlich, dass die Reaktion unerwünschter Weise zum Erliegen kommt. Deshalb muss eine Lauge zugegeben werden, um die entstehenden Protonen, die die Gesamtreaktion bremsen, abzufangen.

Die Art der Lauge, die Konzentration der Lauge und die Geschwindigkeit, mit der die Lauge zugegeben wird, sind entscheidend für die Verteilung der Partikelgrößen der Nanopartikel in der hergestellten Formulierung.

Bevorzugt wird als Base Ammoniak, Kaliumhydrogencarbonat oder Natriumhydroxid eingesetzt. Bei Verwendung dieser Basen werden besonders stabile Dispersionen mit einer engen Verteilung der Partikelgrößen der Nanopartikel erhalten.

Die zugegebene Menge an Lauge ist dabei so zu bemessen, dass nach Abschluss der Reaktion eine pH neutrale Dispersion erhalten wird. Der pH-Wert liegt dann zwischen pH 5 und pH 9.

Laugen bzw. Protonen-Akzeptoren sind definiert durch ihre pKb Werte. Der pKb Wert ist der negative dekadische Logarithmus der Protonenkonzentration im Gleichgewicht und ist somit ein Maß für die Stärke der Base.

Zur Herstellung der Ausgangsformulierung sind Basen geeignet, die einen pKb Wert im Bereich von -2 bis 10,5, bevorzugt 1,5 bis 9,1, besonders bevorzugt im Bereich 3,5 bis 7,5 aufweisen.

Neben der Basenstärke ist zudem die Zugabegeschwindigkeit in die Reaktionslösung zur Herstellung der Formulierung entscheidend. Erfolgt die Zugabe zu schnell, so verschiebt sich das Partikelgrößen-Spektrum hin zu größeren Partikeln, die im Extremfall im Mikrometerbereich liegen. Erfolgt dagegen die Zugabe zu langsam, so werden keine Stoffausbeuten größer 90 % erhalten, da bereits gebildetes Nanosilber katalytisch den Abbau von Reduktionsmitteln hervorruft und damit kein Reaktionspartner mehr zur Erzeugung von Silbernanopartikeln vorliegt.

Experimente haben gezeigt, dass die Zugabegeschwindigkeit der Lauge bei einer Ansatzgröße von 50 kg im Bereich von 9 bis 30 Stunden liegen sollte, um die erfindungsgemäße hohe Qualität an Silbernanopartikeln zu erreichen. Bei entsprechend geringeren Ansatzgrößen verringert sich analog auch die Zeit der Laugenzugabe. Nach oben kann die Zugabezeit nicht beliebig erweitert werden, da der katalytische Abbau des Reduktionsmittels durch bereits entstandenes Nanosilber spätestens nach 48 h die Gesamtausbeute merklich beeinträchtigt.

Die Zugabegeschwindigkeit der Lauge erfolgt so, dass der pH-Wert der Dispersion stets zwischen 0 und 6 liegt. Ein höherer pH-Wert führt zu einer zu schnellen Reaktion und damit zu unkontrolliertem Partikelwachstum. Ein zu niedriger pH-Wert führt dazu, dass die Reaktion zum Erliegen kommt und damit kein Nanosilber mehr gebildet wird.

Insbesondere bei der Herstellung einer nanopartikelhaltigen Mischung durch Zugabe anorganischer Salze kann die durch Zugabe einer Base zu der Lösung von Silbersalz, Stabilisatoren und Reduktionsmittel erhaltene Dispersion in zwei chemische Phasen 1 und 2 getrennt werden. Phase 1 enthält dabei die Silbernanopartikel in dem verwendeten flüssigen Stabilisatorengemisch. Das Lösungsmittel, die anorganischen Salze und das Nebenprodukt Ammoniumnitrat befinden sich in der über der Phase 1 stehenden Phase 2. Die beiden Phasen lassen sich nunmehr auf einfache Weise voneinander separieren, indem die obere Phase 2 abdekantiert wird. Zurück bleibt die Phase 1, die nur mehr aus den Silber-Nanopartikeln und den flüssigen Stabilisatoren besteht.

Die in Form einer Dispersion vorliegenden Silbernanopartikel, die in der Regel Partikelgrößen von 1 nm bis 20 nm aufweisen und chemisch stabilisiert sind, werden auf diese Weise von dem in der Dispersion enthaltenen Nebenprodukt Ammoniumnitrat und dem verwendeten Lösungsmittel, also insbesondere Wasser, abgetrennt.

Besonders gute Ergebnisse werden erzielt, wenn es sich bei dem Lösungsmittel um Wasser und bei den anorganischen Salzen um wasserlösliche anorganische Salze handelt. In diesem Fall wird durch Zugabe wasserlöslicher anorganischer Salze die durch Zugabe einer Base zu der Lösung von Silbersalz, Stabilisator und Reduktionsmittel erhaltene Dispersion in zwei chemische Phasen 1 und 2 getrennt. Phase 1 enthält wiederum die Silbernanopartikel in dem verwendeten flüssigen Stabilisatorengemisch. Das Lösungsmittel Wasser, die wasserlöslichen Salze und das Nebenprodukt Ammoniumnitrat befinden sich in der über der Phase 1 stehenden Phase 2. Die beiden Phasen lassen sich separieren, indem die obere wässrige Phase 2 abdekantiert wird. Zurück bleibt die Phase 1, die nur mehr aus den Silber-Nanopartikeln und den flüssigen Stabilisatoren besteht.

Zur näheren Charakterisierung geeigneter Salze müssen deren kationische und anionische Bestandteile gesondert betrachtet werden. Salze bestehen generell aus mindestens einem Kation und mindestens einem Anion. Eine unerwünschte Wechselwirkung von Kationen mit der stabilisierten silbernanopartikelhaltigen Formulierung ist nicht zu erwarten, da das Silber und insbesondere das bevorzugt verwendete Silber entweder als ungeladenes Silber oder als positiv geladenes Kation vorliegt.

Da als Lösungsmittel Wasser verwendet wird, liegt der Auswahl der geeigneten Kationen/Anionen Kombinationen in Form geeigneter Salze die Vorstellung zugrunde, dass die Phasentrennung hervorgerufen wird durch die Beanspruchung der Hydratationsfähigkeit des Wassers durch geladene Ionen. Die Hydratationsfähigkeit ist dabei ausgedrückt durch die Fähigkeit Wasserstoffbrücken ausbilden zu können. Sie ist zudem der Grund für die Stabilität der Dispersion im Dispersionsmedium Wasser. Damit herrscht also eine gewisse Konkurrenz zwischen den die Silbernanopartikel umhüllenden Stabilisatoren und den gelösten Reaktionsnebenprodukten wie beispielsweise Ammoniumnitrat.

Bei den verwendeten Stabilisatoren handelt es sich um nichtionische Makromoleküle, die lediglich durch schwache Dipol-Dipol-Wechselwirkungen mit den Wassermolekülen in Verbindung stehen. Die Zugabe von Salzen beabsichtigt also den stabilisierenden Einfluss der Wasserstoffbrücken auf die Gesamtdispersion zu entziehen, indem elektrisch geladene Ionen, die wesentlich stärkere Wechselwirkungen mit den Wasserdipolen eingehen, eingebracht werden. In der Regel ist dabei der Grad der Wechselwirkung mit Wasser vom Ionenradius und von der Ionenladung in der Form abhängig, dass die Wechselwirkung mit sinkenden Ionenradien und wachsender Ionenladung zunimmt. Bei der Auswahl geeigneter Salze ist auch deren Löslichkeit in Wasser von Bedeutung. Mit steigender Löslichkeit der Salze in Wasser nimmt auch die Ausbildung von Wechselwirkungskräften mit den Wassermolekülen zu.

Die Auswahl der Anionen ist zudem dadurch eingeschränkt, dass ungewünschte Wechselwirkungen mit vorhandenen Silber-Kationen vermieden werden müssen. Damit scheiden alle Anionen aus, die mit dem eingesetzten Silber und insbesondere mit Silber schwerlösliche Verbindungen bilden, also z.B. Halogenide, Chalkogenide und deren Sauerstoffverbindungen.

Besonders bevorzugt umfasst das anorganische Salz daher zumindest ein Kation der Periode 3 oder der Periode 4 des Periodensystems der Elemente und zumindest ein Element der fünften Hauptgruppe des Periodensystems der Elemente als Bestandteil des Anions, wobei insbesondere bevorzugt das anorganische Salz Stickstoff als Bestandteil des Anions umfasst. Besonders bevorzugt erfolgt ein Abdekantieren der sich nach Zugabe des anorganischen Salzes bildenden Phase von der im Wesentlichen aus Silbernanopartikel und Stabilisatoren bestehenden silbernanopartikelhaltigen Mischung.

### Kurze Beschreibung der Zeichnungen

Bevorzugte Ausführungsformen der Erfindung werden im Folgenden anhand der Zeichnungen beschrieben, die lediglich zur Erläuterung dienen und nicht einschränkend auszulegen sind.

In den Zeichnungen zeigen:
- Fig. 1: ein UVvis-Spektrum einer 5.000-fach verdünnten wässrigen Lösung eines silbernanopartikelhaltigen, dispersen, wässrigen Konzentrats einer Nanosilber-Dispersion mit 10,0 Gew.-% Silber;
- Fig. 2: gemessene Partikelgrößen und berechnete Kurve der Scanning Electron Microscope (SEM)-Datensätze;
- Fig. 3: transmissionselektronenmikroskopische Aufnahme (Transmission Electron Microscope, TEM) Analyse der Silber-Nanopartikel;
- Fig. 4: TEM einer 5.000-fach verdünnten wässrigen Lösung eines silbernanopartikelhaltigen, dispersen, wässrigen Konzentrats.

### Beschreibung bevorzugter Ausführungsformen

### Beispiel 1: Formulierung von Nanosilber mit Hydrazinhydrat, Ammoniak, Tagat TO V^{™} und Tween20^{™}

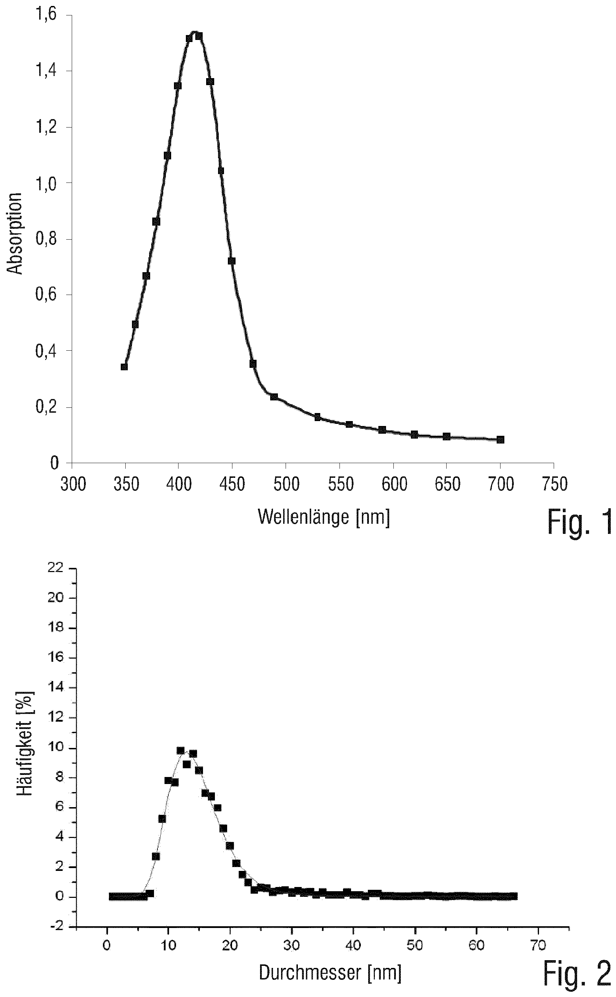

Vorgelegt werden 7.000 g Silbernitrat, 1.760 g Tagat TO V^{™} (nicht-ionisches Polyoxyethylene-25 glycerol trioleat), 1.760 g Tween20^{™} (Polyoxyethylen(20)-sorbitan-monolaurat) und 512 g Hydrazinhydrat in 28.439 g entionisiertem Wasser. Die Lösung wird für 3 Stunden gerührt. Dann werden 5.000 g Ammoniak-Lösung (14 %-ig) über einen Zeitraum von 24 Stunden kontinuierlich zugetropft. Die Reaktion ist nach erfolgter Zugabe abgeschlossen und liefert eine Dispersion mit einem Silbergehalt von 10.0 Gew.-%. Die Partikelgröße und Verteilung wird mittels eines UVvis Spektrums (Figur 1) ermittelt. Im Ergebnis wird eine 10 prozentige Nanosilberdispersion erhalten mit einer Nanosilber-Partikelgröße von 1-20 nm.

Die Methoden zur Bestimmung von Partikelgrößen, z.B. wie oben beschrieben mit TEM sind - bei Nanomaterialien- aufwändig. Deshalb wurde eine indirekte Methode entwickelt, bei der lediglich die Extinktion der Plasmonresonanz der Silbernanopartikel (in Wasser) bestimmt wird, und die mit den Resultaten der TEM Messung für diese Systeme sehr gut korreliert. Das ist eine einfache und schnelle Messung, bei der die Absorption der Silbernanopartikel bei einer Wellenlänge von 350 - 700 nm bestimmt wird.

Die Wellenlänge der maximalen Absorption entspricht der Nano-Silber-Partikelgröße (415 nm z.B. entsprechen einem mittleren Durchmesser von 17 nm bestimmt wie oebn beschrieben mit TEM). Die Peakhöhe korreliert mit der Menge des Nano-Silbers und der Anzahl der Nano-Silberpartikel in der Dispersion.

Das Absorptionsspektrum wird an einer 5.000-fach verdünnten wässrigen Lösung eines silbernanopartikelhaltigen, dispersen, wässrigen Konzentrats der Nanosilber-Dispersion mit 10,0 Gew.-% Silbernanopartikel, durchgeführt, die mithin 20 ppm Nanosilber enthält, klar und tiefgelb gefärbt ist. Das UVvis Spektrum wird im Wellenlängenbereich von 750 nm bis 350 nm aufgenommen. Die gemessenen Absorptionswerte liefern einen Peak mit einem Maximum bei 410 nm - 420 nm und einer Peak-Halbwertsbreite von rund 80 nm.

Die Dispergiereigenschaften der erhaltenen 10 prozentigen Dispersion sind sowohl in polaren als auch in unpolaren Lösungmittel hervorragend, das heißt es wird ohne weiteren chemischen Aufwand (Dispergierhilfen) oder mechanischen Aufwand (Ultraschall, Ultraturax etc.) eine absolut klare Lösung, die lediglich eine durch den Plasmoneffekt des Silbers verursachte Färbung aufweist, erhalten.

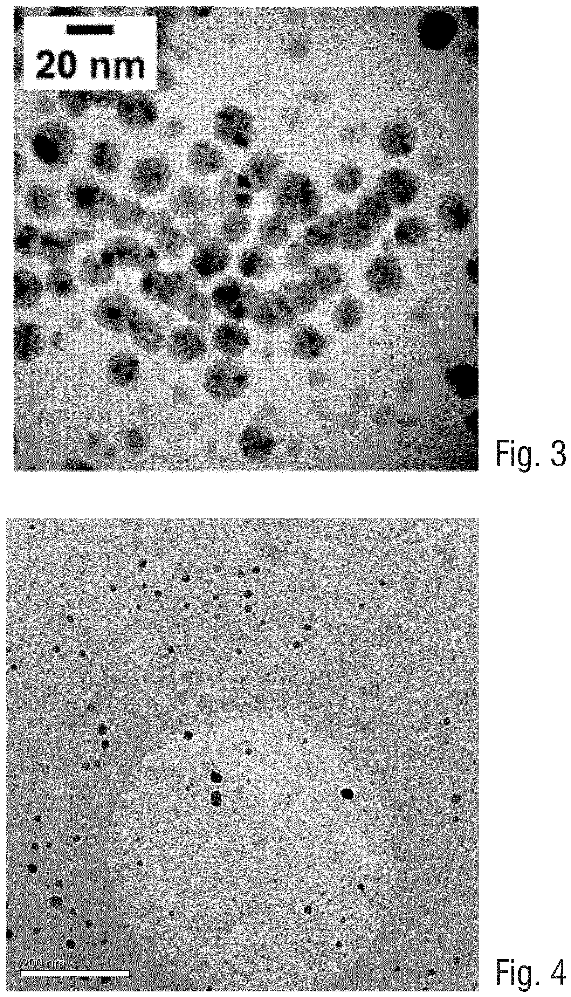

Figur 4 zeigt eine Transmissionselektronenmikroskopische Aufnahme (TEM) der verdünnten Dispersion aus Beispiel 1. Die in der Figur 4 sichtbaren dunklen Bereiche entsprechen den Nano-Silber-Partikeln, die eine Partikelgröße von 1 - 30 nm aufweisen.

### Beispiel 2: Formulierung von Nanosilber mit Hydrazinsulfat, Kaliumhydrogencarbonat, Tagat TO V^{™} und Tween80TM

Vorgelegt werden 7.000 g Silbernitrat, 2.360 g Tagat TO VTM, 1.160 g Tween80^{™} (Polyoxyethylen(80)-sorbitan-monolaurat) und 1.331 g Hydrazinsulfat in 27.620 g entionisiertem Wasser. Die Lösung wird für 3 Stunden gerührt. Dann werden 5.000 g Kaliumhydrogencarbonat-Lösung (1.900 g KHCO₃) über einen Zeitraum von 30 Stunden kontinuierlich zugetropft. Die Reaktion ist nach erfolgter Zugabe abgeschlossen und liefert eine Dispersion mit einem Silbergehalt von 10.0 Gew.-%. Die Partikelgröße und Verteilung wird mittels eines UVvis-Spektrums (analog Figur 1) ermittelt. Im Ergebnis wird eine 10 prozentige Nanosilberdispersion erhalten mit einer Nanosilber-Partikelgröße von 1-30 nm.

### Beispiel 3: Formulierung von Nanosilber mit Glucose, Natriumhydroxid, Tagat L2^{™} (PEG-20 Glyceryl laurat) und Tween20^{™}

Vorgelegt werden 7.000 g Silbernitrat, 2.360 g Tagat L2^{™}, 1.160 g Tween20^{™} und 3.708 g Glucose in 25.243 g entionisiertem Wasser. Die Lösung wird für 3 Stunden gerührt. Dann werden 5.000 g Natriumhydroxid-Lösung (760 g NaOH) über einen Zeitraum von 30 Stunden kontinuierlich zugetropft. Die Reaktion ist nach erfolgter Zugabe abgeschlossen und liefert eine Dispersion mit einem Silbergehalt von 10.0 Gew.-%. Die Partikelgröße und Verteilung wird mittels eines UVvis Spektrums ermittelt. Im Ergebnis wird eine 10 prozentige Nanosilberdispersion erhalten mit einer Nanosilber-Partikelgröße von 1-30 nm.

### Beispiel 4: Weiterverarbeitung der Dispersion aus Beispiel 1 zur Herstellung einer flüssigen, wasser- und salzfreien Formulierung mit Silbernanopartikeln

Die in Beispiel 1 erhaltene Silbernanopartikel enthaltende wässrige Dispersion ist mit dem Nebenprodukt Ammoniumnitrat verunreinigt. Die Silbernanopartikel weisen Partikelgrößen von 1-20 nm auf und sind chemisch stabilisiert. Der Gehalt an Silber beträgt 25 Gew.-%. Der Gehalt an Wasser beträgt 366 g und der Gehalt an Ammoniumnitrat 185 g. Von dieser Dispersion werden 1.000 g in ein Becherglas gegeben und unter Rühren auf 45°C geheizt. Die Trennung der Dispersion in zwei Phasen wird durch Zugabe von 78 g Kaliumnitrat eingeleitet. Nach erfolgter Zugabe und dem vollständigen Auflösen des Kaliumnitrats wird die Heizung entfernt und der Rührer ausgeschaltet. Die Trennung der Phasen kann nach Abkühlung beobachtet werden. Die obere, klare, wässrige Phase 1 wird vollständig abdekantiert. Die zurückbleibende Phase 2 besitzt eine dunkelbraune Farbe mit sirupartiger Fließeigenschaft und einem Gewicht von 449 g.

Eine Analyse der wässrigen Phase 1 ergibt einen Salzgehalt von 263 g und einen Wassergehalt von 366 g.

Die Analyse der silberhaltigen Phase 2 ergibt folgende Daten:
a) Die Analyse des Gesamt-Silbergehaltes durch Veraschung bei 800°C ergibt 250 g Silber. Bezogen auf die Gesamtformulierung entspricht dies einem Silbergehalt von 56 Gew-%.
b) Die Analyse der Partikelgrößenverteilung des erhaltenen Silbers ist in Figur 1 wiedergegeben. Die Bestimmung erfolgt hier durch Messung des UVvis-Absorptionsspektrums im Wellenlängenbereich von 700 nm - 350 nm. Das Peakmaximum bei 415 nm entspricht einer mittleren Partikelgröße von 15 nm. Die Peakhalbwertsbreite von maximal 80 nm ist ein Maß für die enge Partikelgrößenverteilung. Die Korrelation mit den SEM (Scanning Electron Microscope) und den TEM (Transmission Electron Microscope) Analysen, wie in Figur 2 und Figur 3 dargestellt, erlaubt die Angabe der Partikelgrößenverteilung von D99 < 20 nm (99 % der Partikeldurchmesser sind kleiner als 20 nm), sowie als Richtgrössen D33% < 7nm und D66 < 17nm, sowie als Mittelwert einen Wert von ca. 15 nm.

### Beispiel 5: Pilotstudien zur Verwendung einer silbernanopartikelhaltigen, dispersen, wässrigen Formulierung als Medikament zur Behandlung von COVID-19

Als Pilotstudien wurden zwei klinische Studien der Phase 2 (Studie A und Studie B) durchgeführt. Alle Probanden der Studien litten an einer COVID-Pneumonie, die klinisch als mittelschwer bzw. schwer diagnostiziert wurde. Eine solche mittelschwere bis schwere COVID 19-Pneumonie stellt eine ernste, lebensbedrohliche Erkrankung dar, gegen die keine sicher wirksame Behandlungsmethode zur Verfügung steht.

Als Basis für die in den Pilotstudien eingesetzte silbernanopartikelhaltige, disperse, wässrige Formulierung wurde ein Konzentrat (Reinheit der Silbernanopartikel: 99,99%) mit folgender Zusammensetzung eingesetzt:

| **Substanz** | **Anteil [Gew.-%]** |
|---|---|
| Wasser | 74,5 |
| AgNP | 10,0 |
| Ammoniumnitrat | 7,5 |
| Polysorbat 20 | 4,0 |
| PEG 25 Trioleat | 4,0 |

Die eingesetzten Silbernanopartikel AgNP verfügen über folgende Partikelverteilung:
D99: 20 nm; D66 ca. 17 nm; D33 ca. 7 nm. Die Partikelgrössen-Verteilung wurde bestimmt unter Verwendung folgender Methode: Transmissions Electronen Mikroskopie (TEM) wie oben dargelegt.

Polysorbat-20 (CAS-Nummer 9005-64-5) entspricht dem oben genannten Tween 20^{™}, ist ein nichtionisches Tensid und weist einen HLB-Wert von 16,7 auf.

PEG 25 Trioleat ist Peg-25 glyceryl trioleat (CAS 68958-64-5).

Zur Herstellung der in den Pilotstudien eingesetzten silbernanopartikelhaltigen, dispersen, wässrigen Formulierung wurde das obige Konzentrat mit destilliertem Wasser im Verhältnis 1:100 verdünnt. Die erhaltene Formulierung weist folgende Eigenschaften auf:

| | |
|---|---|
| Aggregatszustand: | flüssig |
| Farbe: | bräunlich |
| Geruch: | geruchlos |
| pH-Wert bei 20°C: | 8,0 |
| Dichte: | 1,1 g/cm3 |
| Siedepunkt: | 100°C |
| Konzentration AgNP: | 0,1 Gew.-% |

Die mittlere Größe der Silbernanopartikel liegt um 17 nm (TEM Messungen), D99 < 20nm. Die Nanopartikel weisen eine sphärische Form auf und sind gut wasserlöslich.

### Berechnung der Dosierung

### Inhalation

Der NOAEL-Wert (No Observed Adverse Effect Level) von AgNP für die Sprague Dawley Ratte beträgt 100 µg/m³ [Ji JH, Jung JH, Kim SS, Yoon JU, Park JD, Choi BS, Chung YH, Kwon IH, Jeong J, Han BS, Shin JH. Twenty-eight-day inhalation toxicity study of silver nanoparticles in Sprague-Dawley rats. Inhalation toxicology. 2007 Jan 1;19(10):857-71]. Dies entspricht einem Wert von 59 µg/m3 beim Menschen.

Die in den Pilotstudien vorgesehene Expositionszeit beträgt 8 Stunden pro Tag, die Expositionsdauer 13 Wochen und Häufigkeit der Exposition 65/365. Aus diesen Angaben kann die durchschnittliche zulässige Dosis mit folgender Formel (Exposure Assessment Tools by Routes - Inhalation) berechnet werden: $\begin{matrix}ADD = {C}_{air} \times Inhalationsrate \times Expositionszeit \times Expositionshäufigkeit \times \\ \left(Expositionsdauer/Körpergewicht\right) \times Mittelungszeit \left[Tage\right] \\ = 0.059 \times 32.4 \times 8 \times 0.178 \times \left(0.25/60\right) \times 0.25 \\ = 0 , 003 mg / kg \\ = 3 μg / kg\end{matrix}$

Für eine Expositionszeit von 8 Stunden pro Tag und eine Expositionshäufigkeit von 65 Tagen kann damit die zulässige Gesamtdosis für 13 Wochen berechnet werden:$3 μ g / kg \times 65 = 195 μ g / kg$

### Systemische intravenöse Anwendung

Die systemische Dosis von AgNP für das menschliche Modell kann nach Nair AB, Jacob S. "A simple practice guide for dose conversion between animals and human" Journal of basic and clinical pharmacy. 2016 Mar;7(2):27 berechnet werden.

Für die Human-Äquivalentdosis (HED) gilt: $HED \left(mg/kg\right) = Tier NOAEL \left(mg/kg\right) \times {\left(Gewicht Tier \left[kg\right]/Gewicht Mensch \left[kg\right]\right)}^{\left(1-0,67\right)}$

Unter Berücksichtigung von (siehe: Kim YS, Song MY, Park JD, Song KS, Ryu HR, Chung YH, Chang HK, Lee JH, Oh KH, Kelman BJ, Hwang IK. Subchronic oral toxicity of silver nanoparticles. Particle and fibre toxicology. 2010; 7(1):1-1)
- Referenz-Körpergewicht Maus = 0,02 kg
- Referenz-Körpergewicht Mensch = 60 kg
- NOAEL - 4 mg/kg
erhält man HED = 0.3 mg/kg.

### Forschungsdesign und Methodik

### Fallauswahl

In die Studie A wurden 10 Patienten als Fallgruppe und 10 Patienten als Kontrollgruppe aufgenommen. Alle Patienten litten an einer COVID-Pneumonie, die klinisch als mittelschwer bzw. schwer diagnostiziert wurde. Bei allen Patienten wurde ein Infektion mit der Delta Variante von SARS-CoV-2 festgestellt. Alle Patienten wurden ordnungsgemäß informiert und erteilten ihre Zustimmung.

In die Studie B wurden 10 Patienten als Fallgruppe und 10 Patienten als Kontrollgruppe aufgenommen. Alle Patienten litten an einer COVID-Pneumonie, die klinisch als mittelschwer bzw. schwer diagnostiziert wurde. Bei allen Patienten wurde ein Infektion mit der Omicron Variante von SARS-CoV-2 festgestellt. Alle Patienten wurden ordnungsgemäß informiert und erteilten ihre Zustimmung.

Insgesamt wurden somit 20 Patienten in die Fallgruppe und 20 Patienten in die Kontrollgruppe aufgenommen.

Der Schweregrad der Erkrankung wurde nach dem Early Warning Sign (EWS)-Score basierend auf dem indischen System beurteilt, wobei ein EWS-Score von 5 und höher als schwere Erkrankung bewertet wurde. Der EWS-Score berücksichtigt Alter, Blutdruck, Puls, Atmungsrate und Sauerstoffsättigung. Als Basis kann dabei die Definition dienen, wie sie in "National Early Warning Score (NEWS), Standardising the assessment of acute-illness severity in the NHS, Report of a working party July 2012, Royal College of Physicians, festgelegt ist.

Im spezifischen Zusammenhang mit COVID-19 kann dabei insbesondere auch folgendes Protokoll für die Bestimmung des EWS eingesetzt werden:
National Early Warning Score (NEWS) 2, Standardising the assessment of acute-illness severity in the NHS Additional implementation guidance, Update: March 2020, Royal College of Physicians (https://www.rcplondon.ac.uk/file/20716/download)
Studienart: Offener, randomisierter Vergleich.
Ort der Durchführung der Studie: COVID-Krankenhaus der Tertiärversorgung.

### Einschlusskriterien

RT-PCR bestätigte COVID-19 Patienten beiderlei Geschlechts, Alter über 18 Jahre, mit EWS-Score 5 und höher und klinisch diagnostizierter mittelschwerer bzw. schwerer Pneumonie. Die Patienten mussten die Bereitschaft zur Teilnahme an der Studie erklären.

### Ausschlusskriterien

Patienten mit einem EWS-Score unter 5 und Patienten, die keine Bereitschaft zur Teilnahme an der Studie erklärten.

### Beschreibung der Behandlung

Zunächst wurde die menschliche Äquivalentdosis aus dem NOAEL-Wert (No Observed Adverse Effect Level) berechnet. Als Ergebnis der oben näher erläuterten Berechnung erhält man 0,3 mg/kg. Für einen 60 kg schweren Patienten beträgt die Gesamtdosis 18 mg.

Zu Studienbeginn wurde 1% der Gesamtdosis, also 1,8 mg, für 3 Tage eingesetzt, d.h. 5,4 mg wasserlösliches AgNP. Die Patienten wurden gemäß der oben erläuterten Ein- bzw. Ausschlusskriterien aus dem täglichen Patientenstatusblatt ausgewählt. Die Zustimmung wurde entweder von den Patienten selbst oder von ihren Angehörigen nach einer Beratung über Risiken und Nutzen eingeholt. Insgesamt wurden 20 Patienten ausgewählt und nach dem Zufallsprinzip mittels Blockrandomisierung in eine Fallgruppe und eine Kontrollgruppe eingeteilt.

Die Patienten der Fallgruppe erhielten 1,8 ml des 1:100 verdünnten Konzentrates (Konzentration AgNP: 0,1 Gew.-%) in 500 ml Kochsalzlösung und wurden über 1 Stunde an drei aufeinanderfolgenden Tagen unter Einhaltung aller aseptischen Vorsichtsmaßnahmen infundiert. **In** der Kontrollgruppe wurde kein AgNP verabreicht. Sowohl die Patienten der Fallals auch der Kontrollgruppe erhielten eine Standardbehandlung mit Sauerstoff, Antibiotika, Kortikosteroiden, niedermolekularem Heparin sowie Medikamenten für Begleiterkrankungen wie Insulin für Diabetes und Antihypertonika für Bluthochdruck.

Alle Patienten wurden klinisch im Hinblick auf Bewusstseinslage, Puls, Blutdruck, Sauerstoffsättigung, Atemfrequenz, Urinausscheidung und Infusionsstelle alle 6 Stunden überwacht. Die Ergebnisse und Beobachtungen wurden alle 12 Stunden in einem Patientenstatusblatt festgehalten. Eine routinemäßige Blutuntersuchung wurde am vierten Tag nach Verabreichung der ersten Dosis durchgeführt. Patienten, die einen verbesserten Gesundheitszustand zeigten und hämodynamisch stabil waren, wurden gemäß den Leitlinien entlassen. Die Patienten wurden über einen Zeitraum von 10 Tagen nach der Infusion beobachtet.

### Statistische Methodik

Die Ausgangsvariablen wurden mittels t-Test für kontinuierliche Variablen und Chi-Quadrat-Test (mit anschließendem Wilcoxon-Test) für nominale Variablen in der Gesamtstichprobe verglichen. Die Signifikanz (Alpha) wurde bei p < 0,05 gehalten.

Die Ergebnisvariable (günstiger Verlauf oder entlassen im Gegensatz zu ungünstiger Verlauf oder verstorben) wurde zwischen dem Medikamentenstatus (Fallgruppe vs. Kontrollgruppe) mit dem Chi-Quadrat-Test (mit exakten Fisher-Korrekturen) und dem Wilcoxon-Test verglichen.

### Ergebnisse der Studie A: Delta Variante von SARS-CoV-2

Von den 10 Personen der Kontrollgruppe wurde eine Person während der Nachbeobachtung aus der Analyse ausgeschlossen, da die Person gegen ärztlichen Rat entlassen wurde und ihre Einwilligung zurückzog.

**Tabelle 1A: Fallgruppe mit Alter, EWS-Score zu Beginn, Co-Morbiditäten und Ergebnis**

| Lfd. Nr. | Ausgangswert EWS | Alter [Jahre] | Geschlecht | Co-Morbiditäten | Aktueller Zustand | Dauer des Krankenhausaufenthalts [Tage] |
|---|---|---|---|---|---|---|
| 1 | 15 | 49 | weiblich | Koronare Herzerkrankung mit globaler Ischämie | verstorben | - |
| 2 | 12 | 71 | männlich | Diabetes melitus Typ 2 | verstorben | - |
| 3 | 7 | 52 | weiblich | Diabetes melitus Typ 2 | entlassen | 5 |
| 4 | 9 | 60 | weiblich | Bluthochdruck | entlassen | 5 |
| 5 | 10 | 84 | männlich | keine Co-Morbidität | entlassen | 5 |
| 6 | 8 | 30 | männlich | keine Co-Morbidität | entlassen | 5 |
| 7 | 8 | 72 | männlich | Diabetes melitus Typ 2 | entlassen | 7 |
| 8 | 11 | 62 | männlich | Bluthochdruck | entlassen | 9 |
| 9 | 10 | 79 | männlich | Bluthochdruck | entlassen | 10 |
| 10 | 13 | 62 | männlich | Bluthochdruck, Diabetes melitus Typ 2 | verstorben | - |

**Tabelle 1B: Kontrollgruppe mit Alter, EWS-Score zu Beginn, Co-Morbiditäten und Ergebnis**

| Lfd. Nr. | Ausgangswert EWS | Alter [Jahre] | Geschlecht | Co-Morbiditäten | Aktueller Zustand | Dauer des Krankenhausaufenthalts [Tage] |
|---|---|---|---|---|---|---|
| 1 | 12 | 71 | männlich | Diabetes melitus Typ 2 | ausgeschieden aus der Nachbeobachtung wegen Entlassung auf eigenes Risiko und Rücknahme der Einwilligung | - |
| 2 | 8 | 58 | männlich | keine Co-Morbidität | verstorben | - |
| 3 | 12 | 42 | männlich | Bluthochdruck, Diabetes melitus Typ 2 | verstorben | - |
| 4 | 8 | 68 | männlich | Bluthochdruck | entlassen | 12 |
| 5 | 13 | 74 | männlich | Parkinson | verstorben | - |
| 6 | 12 | 70 | weiblich | Bluthochdruck | verstorben | - |
| 7 | 11 | 40 | männlich | Bluthochdruck | verstorben | - |
| 8 | 7 | 84 | männlich | keine Co-Morbidität | verstorben | - |
| 9 | 8 | 65 | männlich | Diabetes melitus Typ 2 | verstorben | - |
| 10 | 8 | 76 | männlich | Bluthochdruck | verstorben | - |

Ein Vergleich der Tabellen 1A und 1B zeigt einen Trend zu günstigeren Krankheitsverläufen (Entlassung nach klinischem Abklingen) in der adjuvanten AgNP-Gruppe (Fallgruppe) im Vergleich zur Kontrollgruppe mit üblicher Behandlung.

Die Mortalitätsrate betrug bei der Kontrollgruppe 80%, bei der Fallgruppe 30%. Die durchschnittliche Aufenthaltsdauer im Krankenhaus betrug bei der Kontrollgruppe 12 Tage, bei der Fallgruppe 7±2 Tage.

### Ergebnisse der Studie B: Omicron Variante von SARS-CoV-2

**Tabelle 2A: Fallgruppe mit Alter, EWS-Score zu Beginn, Co-Morbiditäten und Ergebnis**

| Lfd. Nr. | Ausgangswert EWS | Alter [Jahre] | Geschlecht | Co-Morbiditäten | Aktueller Zustand | Dauer des Krankenhausaufenthalts [Tage] |
|---|---|---|---|---|---|---|
| 1 | 13 | 70 | männlich | Diabetes melitus Typ 2, Bluthochdruck | verstorben | - |
| 2 | 9 | 75 | männlich | Diabetes melitus Typ 2, Bluthochdruck | entlassen | 7 |
| 3 | 8 | 76 | männlich | Bluthochdruck | entlassen | 8 |
| 4 | 9 | 66 | männlich | Diabetes melitus Typ 2, Bluthochdruck, CKD | verstorben | - |
| 5 | 11 | 80 | männlich | Diabetes melitus Typ 2 | entlassen | 7 |
| 6 | 10 | 83 | männlich | Diabetes melitus Typ 2, Bluthochdruck | entlassen | 6 |
| 7 | 12 | 70 | männlich | Diabetes melitus Typ 2, Bluthochdruck, COPD, CAD | verstorben | - |
| 8 | 10 | 93 | männlich | Diabetes melitus Typ 2, Bluthochdruck | verstorben | - |
| 9 | 10 | 85 | weiblich | Diabetes melitus Typ 2, Bluthochdruck, COPD | entlassen | 7 |
| 10 | 7 | 66 | weiblich | Diabetes melitus Typ 2, Bluthochdruck | entlassen | 12 |

**Tabelle 2B: Kontrollgruppe mit Alter, EWS-Score zu Beginn, Co-Morbiditäten und Ergebnis**

| Lfd. Nr. | Ausgangswert EWS | Alter [Jahre] | Geschlecht | Co-Morbiditäten | Aktueller Zustand | Dauer des Krankenhausaufenthalts [Tage] |
|---|---|---|---|---|---|---|
| 1 | 10 | 68 | weiblich | Diabetes melitus Typ 2, Bluthochdruck | verstorben | - |
| 2 | 8 | 77 | männlich | Diabetes melitus Typ 2 | entlassen | 10 |
| 3 | 7 | 75 | männlich | Diabetes melitus Typ 2, Bluthochdruck | entlassen | 10 |
| 4 | 8 | 78 | weiblich | Diabetes melitus Typ 2 | verstorben | - |
| 5 | 8 | 86 | männlich | Diabetes melitus Typ 2, Bluthochdruck | verstorben | - |
| 6 | 7 | 81 | weiblich | Diabetes melitus Typ 2 | verstorben | - |
| 7 | 9 | 65 | weiblich | Diabetes melitus Typ 2 | entlassen | 18 |
| 8 | 10 | 85 | männlich | Diabetes melitus Typ 2, DVT | verstorben | - |
| 9 | 5 | 56 | männlich | Diabetes melitus Typ 2, Bluthochdruck, CKD | verstorben | - |
| 10 | 12 | 75 | männlich | Diabetes melitus Typ 2, Bluthochdruck | verstorben | - |

Ein Vergleich der Tabellen 2A und 2B zeigt einen Trend zu günstigeren Krankheitsverläufen (Entlassung nach klinischem Abklingen) in der adjuvanten AgNP-Gruppe (Fallgruppe) im Vergleich zur Kontrollgruppe mit üblicher Behandlung.

Die Mortalitätsrate betrug bei der Kontrollgruppe 70%, bei der Fallgruppe 40%. Die durchschnittliche Aufenthaltsdauer im Krankenhaus betrug bei der Kontrollgruppe 13±4 Tage, bei der Fallgruppe 8±2 Tage.

**Tabelle 3: Vergleich der kontinuierlichen Ausgangsvariablen zwischen den Gruppen (Fallgruppe [+]; Kontrollgruppe [-])**

| Demografische Daten | Statistik | Fallgruppe | Kontrollgruppe | Gesamt |
|---|---|---|---|---|
| N | | 20 | 20 | 40 |
| Geschlecht [N[%]] | Männlich | 15 (75%) | 15 (75%) | 30 (75%) |
| | Weiblich | 5 (25%) | 5 (25%) | 10 (25%) |
| Alter [Jahre] | Mittelwert (Standardabweichung) | 69,3 (14,5) | 69,7 (12,8) | 69,5 (13,5) |
| | Median | 70.5 | 72.5 | 71 |
| | Min | 30 | 40 | 30 |
| | Max | 93 | 86 | 93 |
| | Männlich | 10,3 (1,7) | 9,1 (2,4) | |
| EWS | Weiblich | 9,6 (3,3) | 9,2 (1,9) | |
| | Gesamt | 10,1 (2,1) | 9,2 (2,2) | |

Die Werte der Tabelle 3 zeigen, dass die Personen der Fallgruppe und der Kontrollgruppe im Mittel vergleichbares Geschlecht, Alter und EWS-Schweregrad aufwiesen.

**Tabelle 4: Vergleich der nominalen Ausgangsvariablen**

| | | Status | | P (Wilcoxon-Test) |
|---|---|---|---|---|
| | | Fallgruppe | Kontrollgruppe | |
| Geschlecht | männlich | 15 (75%) | 15 (75%) | 1.000 |
| | weiblich | 5 (25%) | 5 (25%) | |
| Co-Morbiditäten | Ja | 19 (95,0%) | 18 (90%) | 0,5483 |
| | Nein | 1 (5,0%) | 2 (10%) | |
| EWS bei Aufnahme in Studie | | 10,1 (2,1) | 9,2 (2,2) | 0,1911 |

Die Werte der Tabelle 4 zeigen, dass es keine statistischen Unterschiede zwischen der Fall- und der Kontrollgruppe in Bezug auf das Alter der Patienten gab. Des Weiteren werden die Ausgangsvariablen der Fallgruppe und der Kontrollgruppe statistisch verglichen.

**Tabelle 5: Vergleich des Erkrankungsverlaufs**

| Ergebnis | | | p |
|---|---|---|---|
| | günstig | ungünstig | |
| Fallgruppe Studie A | 7 (70,0%) | 3 (30,0%) | |
| Kontrollgruppe Studie A | 1 (11,1%) | 8 (88,9%) | |
| Fallgruppe Studie B | 6 (60,0%) | 4 (40,0%) | |
| Kontrollgruppe Studie B | 3 (30,0)% | 7 (70,0%) | 0,0881 |
| Fallgruppe Gesamt | 13 (65,0%) | 7 (35,0%) | |
| Kontrollgruppe Gesamt | 4 (20,0%) | 15 (75,0%) | |

Es zeigt sich ein Trend zu einem günstigeren Ergebnis in der Fallgruppe im Vergleich zu der Kontrollgruppe (p = 0,0881). Dies bedeutet, dass ein ungünstiger Krankheitsverlauf einschließlich Tod des Patienten in der Kontrollgruppe mit höherer Wahrscheinlichkeit gegeben war als in der Fallgruppe.

### Verträglichkeit der Formulierung

Ein Patient der Fallgruppe (Patient Nr. 1) verstarb innerhalb von 12 Stunden nach dem ersten Erhalt der Formulierung. Der Patient hatte eine globale kardiale Ischämie. Es ist unklar, ob dies auf COVID-19 selbst zurückzuführen war. Die Auswirkung von AgNP auf die kardiale Ischämie ist Gegenstand weiterer Untersuchungen. Bei allen anderen Patienten der Fallgruppe kam es zu keiner akuten Verschlechterung des klinischen Status einschließlich der Vitalfunktionen. Es gibt keinen Zusammenhang zwischen dem Tod des Patienten Nr. 1 und der Behandlung mit der Formulierung.

## Patentansprüche

1. Formulierung zur Verwendung als Medikament bei der Prävention und/oder der Behandlung von mikrobiellen und/oder Virus-basierten Erkrankungen des Respirationstraktes,
wobei die Formulierung in Wasser 0,02 Gew.-% bis 0,5 Gew.-% Silbernanopartikel mit einer mittleren Partikelgröße von 10 nm bis 20 nm und wenigstens zwei verschiedene Stabilisatoren aus der Gruppe der nichtionischen Tenside umfasst,
wobei das Gewichtsverhältnis Silbernanopartikel zur Summe der Stabilisatoren im Bereich von 10:2 bis zu 10:50 liegt,
und wobei die wenigstens zwei verschiedenen Stabilisatoren ausgewählt sind aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester, Polyoxypropylen-tri-alkylsäureester und deren Gemische.

2. Formulierung zur Verwendung als Medikament nach Anspruch 1, **dadurch gekennzeichnet, dass** es sich bei der Virus-basierten Erkrankung um akute Rhinits, chronische Rhinitis, Paryngitis, Herpangina, Angina lateralis, Tonsilitis, Laryngitis, Tracheitis, akute Bronchiolitis, chronische Bronchiolitis, akute Bronchitis, chronische Bronchitis, Aveolitis, Pneumonie, akute Sinusitis, chronische Sinusitis, Gingivastomatitis herpetica, Aphtosis herpetica, Herpes nasalis, Adenovirus-Pharyngokonjunktivitis, infektiöse Mononukleose, akutes Asthma, chronisches Asthma, COPD (chronic obstructive pulmonary disease), Kehlkopfdiphterie, Otitis media, Entzündung der Eustachischen Röhre und/oder um COVID-19 handelt, oder dass es sich um eine Kombination von zwei oder mehreren Virus-basierten Erkrankungen des Respirationstraktes handelt, gegebenenfalls in Kombination mit wenigstens einer oder mehreren bakteriellen Sekundärinfektionen
und/oder dass es sich bei der mikrobiellen Erkrankung um eine Pneumokokken, Staphylokokken, Enterobakterien wie insbesondere Klebsiellen oder *Escherichia coli, Pseudomonas aeruginosa, Haemophilus influenzae* und/oder *Mykoplasma pneumoniae* Erkrankung handelt, gegebenenfalls in Kombination mit wenigstens einer oder mehreren viralen Sekundärinfektionen.

3. Formulierung zur Verwendung als Medikament nach einem der Ansprüche 1 oder 2,
**dadurch gekennzeichnet, dass**
es sich bei dem Virus um ein Virus der Familie Picornaviridae, Orthomyxoviridae, Paramyxoviriae, Coronaviridae, Adenoviridae und/oder Herpesviridae handelt oder dass es sich bei dem Virus um ein Virus der Gattung Rhinovirus, Enterovirus, Influenzavirus, Pneumovirus, Metapneumovirus, Betacoronavirus, Mastadenovirus, Simplexvirus und/oder Varizellovirus handelt,
vorzugsweise um ein Virus der Art Rhinivirus, Cosackievirus-1, Cosackievirus-2, Echovirus, Poliovirus, Influenzavirus A, Influenzavirus B, Parainfluenzavirus, Respiratorisches Synzitialvirus (RSV), Humanes Metapneumovirus, severe acute respiratory syndrome-related Coronavirus (SARS-CoV), humanes Adenovirus A, humanes Adenovirus B, humanes Adenovirus C, humanes Adenovirus D, humanes Adenovirus E, humanes Adenovirus F, humanes Herpesvirus 4 (HHV-4), Herpes simplex Virus 1 (HSV-1), Herpes simplex Virus 2 (HSV-2) und/oder Varizella zoster Virus (VZV) handelt.

4. Formulierung zur Verwendung als Medikament nach einem der Ansprüche 1 bis 3, **dadurch gekennzeichnet, dass** es sich bei dem Virus um ein Virus der Unterart severe acute respiratory syndrome Coronavirus 2 (SARS-CoV-2) handelt, wobei die Formulierung zur präventiven und/oder kurativen Behandlung eingesetzt werden kann, vorzugsweise zur Behandlung von Patienten mit einem EWS-Score von wenigstens 3, insbesondere von wenigstens 4.

5. Formulierung zur Verwendung als Medikament nach einem der Ansprüche 1 bis 4, **dadurch gekennzeichnet, dass** die Formulierung zur intravenösen Verabreichung, vorzugsweise zur kontinuierlichen Verabreichung oder zur Bolus-Verabreichung oder zur Verabreichung innerhalb von 5-120 Minuten pro Tag, oder zur Verabreichung per Inhalation als Aerosol geeignet ist.

6. Formulierung zur Verwendung als Medikament nach einem der Ansprüche 1 bis 5,
**dadurch gekennzeichnet, dass** die Silbernanopartikel eine Größenverteilung aufweisen, bei der 99 Gew.-% der Silbernanopartikel kleiner sind als 30 nm, vorzugsweise kleiner sind als 20 nm;
bei der 90 Gew.-% der Silbernanopartikel kleiner sind als 20 nm;
und/oder bei der 66 Gew.-% der Silbernanopartikel kleiner sind als 18 nm;
und/oder bei der 33 Gew.-% der Silbernanopartikel kleiner sind als 15 nm,
vorzugsweise kleiner sind als 10 nm.

7. Formulierung zur Verwendung als Medikament nach einem der Ansprüche 1 bis 6, **dadurch gekennzeichnet, dass** die Silbernanopartikel in einer mittleren Partikelgröße von 15 nm bis 20 nm, vorliegen.

8. Formulierung zur Verwendung als Medikament nach einem der Ansprüche 1 bis 7, **dadurch gekennzeichnet, dass** die Silbernanopartikel in einem Anteil von 0,03 Gew.-% bis 0,4 Gew.-% in der Formulierung enthalten sind, bevorzugt in einem Anteil im Bereich von 0,04 Gew.-% bis 0,3 Gew.-%, besonders bevorzugt in einem Anteil im Bereich von 0,05 Gew.-% bis 0,2 Gew.-%.

9. Formulierung zur Verwendung als Medikament nach einem der Ansprüche 1 bis 8, **dadurch gekennzeichnet, dass** die Stabilisatoren ausgewählt sind aus Polyoxyethylen-Sorbitan-Monolaurat, Polyoxyethylen-Sorbitan-Monopalmitat, Polyoxyethylen-Sorbitan-Monostearat, Polyoxyethylen-Sorbitan-Monooleat, Polyoxyethylen-Sorbitan-Tristearat, Polyoxyethylen-Glyceryl-Trioleat, Polyoxyethylen-Glyceryl-Monolaurat, Polyoxyethylen-Glyceryl-Monooleat, Polyoxyethylen-Glyceryl-Monostearat, Polyoxyethylen-Glyceryl-Monoricinoleat und deren Gemische.

10. Formulierung zur Verwendung als Medikament nach einem der Ansprüche 1 bis 9,
**dadurch gekennzeichnet, dass** wenigstens zwei verschiedenen Stabilisatoren in einem Gewichtsverhältnis im Bereich von 1:1 bis 2:1 anwesend sind,
und/oder dass das Gewichtsverhältnis Silbernanopartikel zur Summe der Stabilisatoren im Bereich von 2:1 bis zu 1:1 liegt.

11. Formulierung zur Verwendung als Medikament nach einem Ansprüche 1 bis 10, **dadurch gekennzeichnet, dass** zwei verschiedene Stabilisatoren anwesend sind, wobei der erste der Stabilisatoren als Polyoxyethylen-mono-alkylsäureester ausgewählt ist, wobei die Anzahl von Polyoxyethylen-Einheiten bevorzugt im Bereich von 15 bis 90 liegt, insbesondere im Bereich von 20 bis 40, wobei der Stabilisator vorzugsweise als Polyoxyethylen-sorbitan-monolaurat ausgewählt ist, wobei die Anzahl von Polyoxyethylen-Einheiten im Bereich von 20 bis40, bevorzugt bei 20 oder 21 liegt, insbesondere bevorzugt ausgewählt als Polyoxyethylen(20)-sorbitan-monolaurat,
und wobei der zweite der Stabilisatoren als Polyoxyethylen-tri-alkylsäureester ausgewählt ist, wobei die Anzahl von Polyoxyethylen-Einheiten bevorzugt im Bereich von 15 bis 60 liegt, insbesondere im Bereich von 20 bis 40, wobei der zweite Stabilisator vorzugsweise als Polyoxyethylen glyceryl trioleat ausgewählt ist, wobei die Anzahl von Polyoxyethylen-Einheiten im Bereich von 20 bis 40, bevorzugt bei 30 liegt, insbesondere bevorzugt ausgewählt als Polyoxyethylen (25) glyceryl trioleat,
wobei vorzugsweise das Gewichtsverhältnis vom ersten zum zweiten Stabilisator im Bereich von 2:1-1:2 liegt, vorzugsweise im Bereich von 1:1.

12. Formulierung zur Verwendung als Medikament nach einem der Ansprüche 1 bis 11,
**dadurch gekennzeichnet, dass** die Formulierung in Kombination mit mindestens einem weiteren pharmazeutischen Wirkstoff und/oder mindestens einem weiteren nicht-pharmazeutischen Wirkstoff vorliegt,
wobei der mindestens eine weitere pharmazeutische Wirkstoff vorzugsweise ausgewählt ist aus Virostatika, Sympathomimetika, Proteinen, Peptiden, Nukleinsäuren und immunsuppressiven Wirkstoffen und
wobei der mindestens eine weitere nicht-pharmazeutische Wirkstoff ausgewählt ist aus pharmazeutisch verträglichen anorganischen oder organischen Säuren oder Basen, Polymeren, Copolymeren, Block-Copolymeren, Einfachzucker, Mehrfachzucker, ionischen und nicht-ionischen Tensiden oder Lipiden sowie Mischungen hiervon, Albumin, Transferrin und DNA-Reparatur-Proteinen, wie Kinase-Inhibitoren.

13. Formulierung zur Verwendung als Medikament nach einem der Ansprüche 1 bis 12, **dadurch gekennzeichnet, dass** die Formulierung Ammoniumnitrat in einem Anteil von 0,025 Gew.-% bis 2,0 Gew.-%, bevorzugt 0,05 Gew.-% bis 1,0 Gew.-%, besonders bevorzugt rund 0,075 Gew.-%, pro 0,1 Gew.-% Silbernanopartikel enthält.

## Claims

1. Formulation for use as a medicament in the prevention and/or treatment of microbial and/or viral diseases of the respiratory tract,
wherein the formulation comprises, in water, 0.02% by weight to 0.5% by weight of silver nanoparticles with an average particle size of 10 nm to 20 nm and at least two different stabilisers from the group of non-ionic surfactants,
wherein the weight ratio of silver nanoparticles to the sum of the stabilisers is in the range of 10:2 to 10:50,
and wherein the at least two different stabilisers are selected from polyoxyethylene monoalkyl acid esters, polyoxypropylene monoalkyl acid esters, polyoxyethylene dialkyl acid esters, polyoxypropylene dialkyl acid esters, polyoxyethylene trialkyl acid esters, polyoxypropylene trialkyl acid esters and mixtures thereof.

2. Formulation for use as a medicament according to claim 1, **characterised in that** the virus-based disease is acute rhinitis, chronic rhinitis, pharyngitis, herpangina, lateral angina, tonsillitis, laryngitis, tracheitis, acute bronchiolitis, chronic bronchiolitis, acute bronchitis, chronic bronchitis, alveolitis, pneumonia, acute sinusitis, chronic sinusitis, herpetic gingivostomatitis, herpetic aphthous stomatitis, herpes nasalis, adenoviral pharyngoconjunctivitis, infectious mononucleosis, acute asthma, chronic asthma, COPD (chronic obstructive pulmonary disease), laryngeal diphtheria, otitis media, inflammation of the Eustachian tube and/or COVID-19, or that it involves a combination of two or more virus-based respiratory tract diseases, possibly in combination with at least one or more secondary bacterial infections
and/or that the microbial disease is a pneumococcal, staphylococcal, enterobacterial (such as, in particular, Klebsiella or *Escherichia coli), Pseudomonas aeruginosa, Haemophilus influenzae* and/or *Mycoplasma pneumoniae* infection, possibly in combination with at least one or more secondary viral infections.

3. Formulation for use as a medicament according to any one of claims 1 or 2, **characterised in that**
the virus is a virus of the family Picornaviridae, Orthomyxoviridae, Paramyxoviridae, Coronaviridae, Adenoviridae and/or Herpesviridae
or that the virus is a virus of the genus Rhinovirus, Enterovirus, Influenzavirus, Pneumovirus, Metapneumovirus, Betacoronavirus, Mastadenovirus, Simplexvirus and/or Varizellovirus,
preferably a virus of the species Rhinovirus, Cosackievirus-1, Cosackievirus-2, Echovirus, Poliovirus, Influenza virus A, influenza virus B, parainfluenza virus, respiratory syncytial virus (RSV), human metapneumovirus, severe acute respiratory syndrome-related coronavirus (SARS-CoV), human adenovirus A, human adenovirus B, human adenovirus C, human adenovirus D, human adenovirus E, human adenovirus F, human herpesvirus 4 (HHV-4), herpes simplex virus 1 (HSV-1), herpes simplex virus 2 (HSV-2) and/or varicella-zoster virus (VZV).

4. Formulation for use as a medicament according to any one of claims 1 to 3,
**characterised in that** the virus is a virus of the subspecies severe acute respiratory syndrome coronavirus 2 (SARS-CoV-2), wherein the formulation can be used for preventive and/or curative treatment, preferably for the treatment of patients with an EWS score of at least 3, in particular of at least 4.

5. Formulation for use as a medicament according to any one of claims 1 to 4,
**characterised in that** the formulation is suitable for intravenous administration,
preferably for continuous administration or for bolus administration or for administration within 5-120 minutes per day, or for administration by inhalation as an aerosol.

6. Formulation for use as a medicament according to any one of claims 1 to 5,
**characterised in that** the silver nanoparticles have a size distribution such that 99% by weight of the silver nanoparticles are smaller than 30 nm, preferably smaller than 20 nm;
in which 90% by weight of the silver nanoparticles are smaller than 20 nm;
and/or in which 66% by weight of the silver nanoparticles are smaller than 18 nm;
and/or in which 33% by weight of the silver nanoparticles are smaller than 15 nm,
preferably smaller than 10 nm.

7. Formulation for use as a medicament according to any one of claims 1 to 6,
**characterised in that** the silver nanoparticles are present in a mean particle size of 15 nm to 20 nm.

8. Formulation for use as a medicament according to any one of claims 1 to 7,
**characterised in that** the silver nanoparticles are present in the formulation in an amount of 0.03% by weight to 0.4% by weight, preferably in an amount in the range of 0.04% by weight to 0.3% by weight, particularly preferably in an amount in the range of 0.05% by weight to 0.2% by weight.

9. Formulation for use as a medicament according to any one of claims 1 to 8,
**characterised in that** the stabilisers are selected from polyoxyethylene sorbitan monolaurate, polyoxyethylene sorbitan monopalmitate, polyoxyethylene sorbitan monostearate, polyoxyethylene sorbitan monooleate, polyoxyethylene sorbitan tristearate, polyoxyethylene glyceryl trioleate, polyoxyethylene glyceryl monolaurate, polyoxyethylene glyceryl monooleate, polyoxyethylene glyceryl monostearate, polyoxyethylene glyceryl monoricinoleate and mixtures thereof.

10. Formulation for use as a medicament according to any one of claims 1 to 9,
**characterised in that** at least two different stabilisers are present in a weight ratio in the range of 1:1 to 2:1,
and/or that the weight ratio of silver nanoparticles to the sum of the stabilisers lies in the range from 2:1 to 1:1.

11. Formulation for use as a medicament according to any one of claims 1 to 10,
**characterised in that** two different stabilisers are present, wherein the first of the stabilisers is selected as a polyoxyethylene monoalkyl acid ester, wherein the number of polyoxyethylene units is preferably in the range of 15 to 90, in particular in the range of 20 to 40, wherein the stabiliser is preferably selected as polyoxyethylene sorbitan-monolaurate, wherein the number of polyoxyethylene units lies in the range of 20 to 40, preferably at 20 or 21, and is particularly preferably selected as polyoxyethylene(20)-sorbitan-monolaurate,
and wherein the second of the stabilisers is selected as a polyoxyethylene trialkyl acid ester, wherein the number of polyoxyethylene units is preferably in the range of 15 to 60, particularly in the range of 20 to 40, wherein the second stabiliser is preferably selected as polyoxyethylene glyceryl trioleate, wherein the number of polyoxyethylene units is in the range of 20 to 40, preferably 30, and is particularly preferably selected as polyoxyethylene (25) glyceryl trioleate,
wherein the weight ratio of the first to the second stabiliser is preferably in the range of 2:1 to 1:2, preferably in the range of 1:1.

12. Formulation for use as a medicament according to any one of claims 1 to 11,
**characterised in that** the formulation is present in combination with at least one further active pharmaceutical ingredient and/or at least one further non-pharmaceutical ingredient,
wherein the at least one further active pharmaceutical ingredient is preferably selected from antivirals, sympathomimetics, proteins, peptides, nucleic acids and immunosuppressive agents, and
wherein the at least one further non-pharmaceutical active ingredient is selected from pharmaceutically acceptable inorganic or organic acids or bases, polymers, copolymers, block copolymers, monosaccharides, polysaccharides, ionic and non-ionic surfactants or lipids, as well as mixtures thereof, albumin, transferrin and DNA repair proteins, such as kinase inhibitors.

13. Formulation for use as a medicament according to any one of claims 1 to 12, **characterised in that** the formulation contains ammonium nitrate in a proportion of 0.025% by weight to 2.0% by weight, preferably 0.05% by weight to 1.0% by weight, more preferably around 0.075% by weight, per 0.1% by weight of silver nanoparticles.

## Revendications

1. Formulation destinée à être utilisée comme médicament dans la prévention et/ou le traitement de maladies microbiennes et/ou virales des voies respiratoires,
la formulation comprenant, dans l'eau, de 0,02 % en poids à 0,5 % en poids de nanoparticules d'argent ayant une taille des particules moyenne de 10 nm à 20 nm et au moins deux stabilisants différents choisis parmi le groupe des tensioactifs non ioniques,
dans lequel le rapport pondéral entre les nanoparticules d'argent et la somme des stabilisants se situe dans la plage de 10:2 à 10:50,
et dans lequel les au moins deux stabilisants différents sont choisis parmi d'ester d'acide monoalkylique de polyoxyéthylène, d'ester d'acide monoalkylique de polyoxypropylène, d'ester d'acide dialkylique de polyoxyéthylène, d'ester d'acide dialkylique de polyoxypropylène, d'ester d'acide trialkylique de polyoxyéthylène, d'ester d'acide trialkylique de polyoxypropylène et de leurs mélanges.

2. Formulation destinée à être utilisée comme médicament selon la revendication 1, **caractérisée en ce que** la maladie d'origine virale est une rhinite aiguë, une rhinite chronique, une pharyngite, une herpangine, une angine latérale, une amygdalite, une laryngite, une trachéite, une bronchiolite aiguë, une bronchiolite chronique, une bronchite aiguë, une bronchite chronique, une alvéolite, une pneumonie, une sinusite aiguë, une sinusite chronique, une gingivostomatite herpétique, une aphtose herpétique, un herpès nasal, une pharyngoconjonctivite à adénovirus, une mononucléose infectieuse, de l'asthme aigu, de l'asthme chronique, une BPCO (bronchopneumopathie chronique obstructive), diphtérie laryngée, otite moyenne, inflammation de la trompe d'Eustache et/ou de la COVID-19, ou qu'il s'agit d'une combinaison de deux ou plusieurs maladies virales des voies respiratoires, le cas échéant en association avec au moins une ou plusieurs infections bactériennes secondaires
et/ou que la maladie microbienne est une infection à pneumocoques, staphylocoques, entérobactéries telles que notamment les Klebsiella ou *Escherichia coli, Pseudomonas aeruginosa, Haemophilus influenzae* et/ou *Mycoplasma pneumoniae,* le cas échéant en association avec au moins une ou plusieurs infections virales secondaires.

3. Formulation destinée à être utilisée comme médicament selon l'une des revendications 1 ou 2, **caractérisée en ce que**
le virus est un virus de la famille des Picornaviridae, Orthomyxoviridae, Paramyxoviridae, Coronaviridae, Adenoviridae et/ou Herpesviridae ou que le virus est un virus du genre Rhinovirus, Enterovirus, Influenzavirus, Pneumovirus, Metapneumovirus, Betacoronavirus, Mastadenovirus, Simplexvirus et/ou Varizellovirus,
de préférence un virus de l'espèce Rhinivirus, Cosackievirus-1, Cosackievirus-2, Echovirus, Poliovirus, Influenzavirus A, Influenzavirus B, parainfluenzavirus, virus respiratoire syncytial (VRS), métapneumovirus humain, coronavirus lié au syndrome respiratoire aigu sévère (SARS-CoV), adénovirus humain A, adénovirus humain B, adénovirus humain C, adénovirus humain D, adénovirus humain E, adénovirus humain F, l'herpèsvirus humain 4 (HHV-4), le virus de l'herpès simplex 1 (HSV-1), le virus de l'herpès simplex 2 (HSV-2) et/ou le virus varicelle-zoster (VZV).

4. Formulation destinée à être utilisée comme médicament selon l'une des revendications 1 à 3, **caractérisée en ce que** le virus est un virus de la sous-espèce coronavirus du syndrome respiratoire aigu sévère 2 (SARS-CoV-2), dans lequel la formulation peut être utilisée pour un traitement préventif et/ou curatif, de préférence pour le traitement de patients présentant un score EWS d'au moins 3, en particulier d'au moins 4.

5. Formulation destinée à être utilisée comme médicament selon l'une des revendications 1 à 4, **caractérisée en ce que** la formulation est adaptée à une administration intraveineuse, de préférence à une administration continue ou à une administration en bolus ou à une administration sur une durée de 5 à 120 minutes par jour, ou à une administration par inhalation sous forme d'aérosol.

6. Formulation destinée à être utilisée comme médicament selon l'une des revendications 1 à 5, **caractérisée en ce que** les nanoparticules d'argent présentent une distribution granulométrique telle que 99 % en poids des nanoparticules d'argent ont une taille inférieure à 30 nm, de préférence inférieure à 20 nm;
dans laquelle 90 % en poids des nanoparticules d'argent ont une taille inférieure à 20 nm;
et/ou dans laquelle 66 % en poids des nanoparticules d'argent ont une taille inférieure à 18 nm;
et/ou dans laquelle 33 % en poids des nanoparticules d'argent ont une taille inférieure à 15 nm, de préférence inférieure à 10 nm.

7. Formulation destinée à être utilisée comme médicament selon l'une des revendications 1 à 6, **caractérisée en ce que** les nanoparticules d'argent ont une taille des particules moyenne comprise entre 15 nm et 20 nm.

8. Formulation destinée à être utilisée comme médicament selon l'une des revendications 1 à 7, **caractérisée en ce que** les nanoparticules d'argent sont contenues dans la formulation en une proportion de 0,03 % en poids à 0,4 % en poids, de préférence en une proportion dans la plage de 0,04 % en poids à 0,3 % en poids, en particulier de préférence en une proportion dans la plage de 0,05 % en poids à 0,2 % en poids.

9. Formulation destinée à être utilisée comme médicament selon l'une des revendications 1 à 8, **caractérisée en ce que** les stabilisants sont choisis parmi de monolaurate de polyoxyéthylène-sorbitane, de monopalmitate de polyoxyéthylène-sorbitane, de monostéarate de polyoxyéthylène-sorbitane, de monooléate de polyoxyéthylène-sorbitane, de tristearate de polyoxyéthylène-sorbitane, de trioléate de polyoxyéthylène-glycéryle, de monolaurate de polyoxyéthylène-glycéryle, de monooléate de polyoxyéthylène-glycéryle, de monostéarate de polyoxyéthylène-glycéryle, de mono-ricinoléate de polyoxyéthylène-glycéryle et leurs mélanges.

10. Formulation destinée à être utilisée comme médicament selon l'une des revendications 1 à 9, **caractérisée en ce qu'**au moins deux stabilisants différents sont présents dans un rapport pondéral dans une plage de 1:1 à 2:1,
et/ou **en ce que** le rapport pondéral entre les nanoparticules d'argent et la somme des stabilisants se situe dans une plage de 2:1 à 1:1.

11. Formulation destinée à être utilisée comme médicament selon l'une des revendications 1 à 10, **caractérisée en ce que** deux stabilisants différents sont présents, le premier des stabilisants étant choisi comme un ester d'acide monoalkylique de polyoxyéthylène, le nombre d'unités de polyoxyéthylène se situant de préférence dans la plage de 15 à 90, en particulier dans la plage de 20 à 40, dans lequel le stabilisant est de préférence choisi comme un monolaurate de polyoxyéthylène-sorbitane-monolaurate, le nombre d'unités de polyoxyéthylène se situant dans la plage de 20 à 40, de préférence à 20 ou 21, et étant de préférence choisi comme polyoxyéthylène(20)-sorbitan-monolaurate,
et dans lequel le deuxième des stabilisants est choisi comme un ester d'acide tri alkylique de polyoxyéthylène, le nombre d'unités de polyoxyéthylène se situant de préférence dans la plage de 15 à 60, en particulier dans la plage de 20 à 40, dans lequel le deuxième stabilisant est de préférence choisi comme trioléate de glycéryle de polyoxyéthylène, le nombre d'unités de polyoxyéthylène se situant dans la plage de 20 à 40, de préférence à 30, et est en particulier de préférence choisi comme trioléate de glycéryle de polyoxyéthylène (25) ,
dans lequel le rapport pondéral entre le premier et le deuxième stabilisant se situe de préférence dans la plage de 2:1 à 1:2, de préférence dans la plage de 1:1.

12. Formulation destinée à être utilisée comme médicament selon l'une des revendications 1 à 11, **caractérisée en ce que** la formulation se présente en combinaison avec au moins un autre principe actif pharmaceutique et/ou au moins un autre principe actif non pharmaceutique,
Dans lequel l'au moins un autre principe actif pharmaceutique est de préférence choisi parmi des antiviraux, des sympathomimétiques, des protéines, des peptides, des acides nucléiques et des agents immunosuppresseurs,
et dans lequel ledit au moins un autre principe actif non pharmaceutique est choisi parmi des acides ou bases inorganiques ou organiques pharmaceutiquement acceptables, des polymères, des copolymères, des copolymères à blocs, des monosaccharides, des polysaccharides, des tensioactifs ioniques et non ioniques ou des lipides, ainsi que leurs mélanges, d'albumine, de transferrine et des protéines de réparation de l'ADN, telles que des inhibiteurs de kinase.

13. Formulation destinée à être utilisée comme médicament selon l'une des revendications 1 à 12, **caractérisée en ce que** la formulation contient du nitrate d'ammonium en une proportion de 0,025 % en poids à 2,0 % en poids, de préférence de 0,05 % en poids à 1,0 % en poids, de manière particulièrement préférée d'environ 0,075 % en poids, par 0,1 % en poids de nanoparticules d'argent.
